(19) Europäisches Patentamt
European Patent Office
Office européen des brevets

(11) EP 3 794 976 A1

(12) EUROPEAN PATENT APPLICATION

(43) Date of publication:
24.03.2021 Bulletin 2021/12

(51) Int Cl.:
A24F 40/10 (2020.01)          A24F 40/40 (2020.01)
A61M 15/00 (2006.01)

(21) Application number: 19198636.3

(22) Date of filing: 20.09.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: NERUDIA LIMITED
Liverpool Merseyside L24 9HP (GB)

(72) Inventor: The designation of the inventor has not
yet been filed

(74) Representative: Mewburn Ellis LLP
Aurora Building
Counterslip
Bristol BS1 6BX (GB)

(54) SMOKING SUBSTITUTE APPARATUS

(57)    A smoking substitute apparatus for generating an aerosol, comprising: a housing; an air inlet and an outlet formed at the housing; a passage extending between the air inlet and the outlet, in use an air flow entering into the housing to flow along the passage to the outlet for inhalation by a user drawing on the apparatus; an aerosol generation chamber containing an aerosol generator being operable to generate an aerosol from an aerosol precursor; the aerosol generation chamber comprises at least one chamber outlet in fluid communication with the passage, the at least one chamber outlet permitting, in use, aerosol generated by the aerosol generator to be entrained into the air flow along the passage; and a bleed aperture opening into the aerosol generation chamber and configured to allow air to enter the aerosol generation chamber in an amount that contributes not more than 35% of the air flow at the outlet for inhalation by a user drawing on the apparatus.

FIG. 21A

FIG. 21B

FIG. 21C

**Description**

*Field of the Invention*

The present invention relates to a smoking substitute apparatus and, in particular, a smoking substitute apparatus that is able to reduce fluid leakage during use, as well as to deliver nicotine to a user in an effective manner.

*Background*

The smoking of tobacco is generally considered to expose a smoker to potentially harmful substances. It is thought that a significant amount of the potentially harmful substances are generated through the burning and/or combustion of the tobacco and the constituents of the burnt tobacco in the tobacco smoke itself.

Low temperature combustion of organic material such as tobacco is known to produce tar and other potentially harmful by-products. There have been proposed various smoking substitute systems in which the conventional smoking of tobacco is avoided.

Such smoking substitute systems can form part of nicotine replacement therapies aimed at people who wish to stop smoking and overcome a dependence on nicotine.

Known smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the health risks associated with conventional smoking.

In general, smoking substitute systems are intended to provide a substitute for the rituals of smoking, whilst providing the user with a similar, or improved, experience and satisfaction to those experienced with conventional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach. Some smoking substitute systems are designed to resemble a conventional cigarette and are cylindrical in form with a mouthpiece at one end. Other smoking substitute devices do not generally resemble a cigarette (for example, the smoking substitute device may have a generally box-like form, in whole or in part).

One approach is the so-called "vaping" approach, in which a vaporisable liquid, or an aerosol former or aerosol precursor, sometimes typically referred to herein as "e-liquid", is heated by a heating device (sometimes referred to herein as an electronic cigarette or "e-cigarette" device) to produce an aerosol vapour which is inhaled by a user. The e-liquid typically includes a base liquid, nicotine and may include a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid and a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically couplable to a consumable including the tank and the heating element. In this way, when the tank of a consumable has been emptied of e-liquid, that consumable is removed from the main body and disposed of. The main body can then be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece and a sealed tank which contains e-liquid. The consumable having an inlet which is fluidly connected to an outlet at the mouthpiece by an air flow channel. The consumable further includes a heater, which for this device is a heating filament coiled around a portion of a wick positioned across the width of the air flow passage. The wick is partially immersed in the e-liquid, and conveys e-liquid from the tank to the heating filament. The system is controlled by a microprocessor on board the main body. The system includes a sensor for detecting when a user is inhaling through the mouthpiece, the microprocessor then activating the device in response. When the system is activated, electrical energy is supplied from the power source to the heating device, which heats e-liquid from the tank to produce a vapour, which promptly condenses to form an aerosol as it is cooled by an airflow, or air flow, passing through the air flow passage. A user may therefore inhale the generated aerosol through the mouthpiece.

### Summary of the Invention

For a smoking substitute system it is desirable to deliver nicotine into the user's lungs, where it can be absorbed into the bloodstream. However, the present disclosure is based in part on a realisation that some prior art smoking substitute systems, such delivery of nicotine is not efficient. In some prior art systems, the aerosol droplets have a size distribution that is not suitable for delivering nicotine to the lungs. Aerosol droplets of a large particle size tend to be deposited in the mouth and/or upper respiratory tract. Aerosol particles of a small (e.g. sub-micron) particle size can be inhaled into the lungs but may be exhaled without delivering nicotine to the lungs. As a result the user would require drawing a longer puff, more puffs, or vaporising e-liquid with a higher nicotine concentration in order to achieve the desired experience.

Accordingly, there is a need for improvement in the delivery of nicotine to a user in the context of a smoking substitute system.

The present disclosure has been devised in the light of the above considerations.

In a general aspect, the present invention relates to a smoking substitute apparatus having an aerosol generation chamber with a bleed aperture for allowing a minority air flow to ingress into the aerosol generation chamber.

According to a first preferred aspect there is provided a smoking substitute apparatus for generating an aerosol, comprising:

a housing;

an air inlet and an outlet formed at the housing;

a passage extending between the air inlet and the outlet, in use an air flow entering into the housing to flow along the passage to the outlet for inhalation by a user drawing on the apparatus;

an aerosol generation chamber containing an aerosol generator being operable to generate an aerosol from an aerosol precursor; the aerosol generation chamber comprises at least one chamber outlet in fluid communication with the passage, the at least one chamber outlet permitting, in use, aerosol generated by the aerosol generator to be entrained into the air flow along the passage; and

a bleed aperture opening into the aerosol generation chamber and configured to allow air to enter the aerosol generation chamber in an amount that contributes not more than 35% of the air flow at the outlet for inhalation by a user drawing on the apparatus.

For the avoidance of doubt, we note here that a user may draw on the apparatus with or without making physical contact with the apparatus. For example, a mouthpiece or other intervening structure may be provided, separate from and/or separable from the housing of the smoking substitute apparatus, and the user's lips may make contact with this mouthpiece or other intervening structure when drawing on the apparatus.

The aerosol generation chamber may be provided at or towards a first end of the housing. For example the base of the aerosol generation chamber may form the base of the housing. Said first end of the housing may be engageable with a main body of a smoking substitute system. For convenience, the first end of the housing may considered to be directed towards a downward direction. The outlet may open at a second end of the housing having a mouthpiece which the user may puff onto in order to draw an air flow through the passage. The air inlet may open at a sidewall of the housing, wherein the passage may extend directly towards the outlet at the second end of the housing, or it may extend towards the first end of the housing before routing back towards the outlet at the second end of the housing.

The aerosol precursor may comprise a liquid aerosol precursor, and wherein the aerosol generator may comprise a heater configured to generate the aerosol by vaporising the liquid aerosol precursor. The liquid aerosol precursor may be an e-liquid and may comprise nicotine and a base liquid such as propylene glycol and/or vegetable glycerine and may include a flavourant. The aerosol generator may be a heater such as a heater coil wounded around a wick.

In use, the aerosol generator may vaporise an aerosol precursor to form a vapour. The vapour may expand or flow from the aerosol generator to merge or entrain into the air flow in the passage. Said vapour may cool and condense to from an aerosol in the aerosol generation chamber and subsequently be discharged towards the outlet. More specifically, the chamber outlet may be positioned downstream of aerosol generator along the direction of aerosol flow, and therefore the aerosol in the aerosol generation chamber may discharge towards the outlet through the chamber outlet.

In contrast to prior art smoking substitute systems, which generate an aerosol by passing over the heater an entire amount of air flow that is drawn through the outlet, the passage according to the present disclosure may be routed to allow a significant amount of air flow, e.g. a larger portion of air flow entering the housing, to bypass the aerosol generation chamber and thus the aerosol generator. In other words, the passage may extend externally to the aerosol generation chamber and configured to be in fluid communication with the chamber outlet. More specifically, the passage may be configured to allow the majority of air flow (i.e. a first air flow) in an amount that contributes at least 65% of the air flow to bypass the aerosol generator and only come into contact with the aerosol once the aerosol has been discharged from the aerosol generation chamber. For example, the passage may extend along the external sidewalls of the aerosol generation chamber such that the majority of air flow may flow externally and parallel to the aerosol generation chamber, wherein the aerosol generated in the aerosol generation chamber may be drawn into the first air flow along the passage through the chamber outlet. Furthermore, as the aerosol precursor is being vaporised in the aerosol generation chamber, the vapour may expand in the aerosol generation chamber and thereby it may increase the internal pressure at the aerosol generation chamber. Such elevated internal pressure may advantageously aid the convection of the vapour and/or aerosol towards the chamber outlet.

However, due to a lack of air flow in such "stagnant" volume in the aerosol generation chamber, some generated aerosol produced during a puff may not be able to promptly evacuate from the chamber. For example, as the suction downstream to the chamber outlet ceases at the end of a puff, aerosol that has already been generated in the aerosol generation chamber may not be promptly evacuated through the chamber outlet and may remain in the aerosol generation chamber. Therefore gradually, the aerosol droplets may coalesce and settle, and may adhere onto the sidewall and the base of the aerosol generation chamber. Furthermore, at the end of a puff, a partial vacuum, e.g. a lowered pressure or unequalised pressure, within the aerosol generation chamber may cause the aerosol that has already purged through the chamber outlet to backflow into the chamber, until the pressure in the aerosol generation chamber has been equalised.

Therefore advantageously, a bleed aperture may enable a smaller amount of air flow to enter or bleed into the aerosol generation chamber. The bleed aperture may be configured to allow air (i.e. a second air flow) to enter the aerosol generation chamber in an amount that contributes not more than 35% of the air flow at the outlet. The other air flow, that is the air flow entering into the housing to flow along the passage, may be designated as the first air flow. Together the first air flow and the second air flow may be substantially equal to the total amount of air flow at the outlet. The first air flow and the second air flow may enter the smoking substitute apparatus through the same or different air inlets. Optionally, said second air flow may contribute to less than any one of 30%, 25%, 20%, 15%, 10% and 5% of air flow at the outlet. This may induce a relatively low level of convection and turbulence in the aerosol generation chamber, and yet allowing the generated aerosol to be discharged through the chamber outlet more effectively. For example, in use the second air flow entering through the bleed aperture may flush the aerosol generation chamber. Said second air flow may induce a degree of turbulence in the aerosol generation chamber. However, because of the much reduced amount of air flow, the turbulence in the vicinity of the aerosol generator in the present disclosure may be expected to be significantly lower than the amount of turbulence that is otherwise expected in prior art consumables.

Furthermore, such arrangement may reduce the amount of aerosol being drawn, through the chamber outlet, back into the aerosol generation chamber at the end of a puff. Instead, at the end of the puff, the partial vacuum or lowered pressure in the aerosol generation chamber may cause the second air flow, substantially free of aerosol, to be drawn into the chamber through the bleed aperture.

Optionally, the bleed aperture is configured to have a smaller hydraulic diameter to that of the chamber outlet. Optionally, the bleed aperture has a hydraulic diameter of at least 0.01mm, at least 0.1mm or at least 0.3mm. The bleed aperture may have a hydraulic diameter of up to 10mm, up to 1mm, or up to 0.7mm. Advantageously, the bleed aperture may form a flow constrictor for reducing or limiting the amount of air flow entering the aerosol generation chamber there through during a user puff. That is, the bleed aperture may be sized, with respect to the flow resistance across the passage, to vary the amount of second air flow entering the aerosol generation chamber through the bleed aperture.

Optionally, the chamber outlet and the bleed aperture open at opposing ends of the aerosol generation chamber. For example, the chamber outlet may open towards the outlet of the housing, whereby the bleed aperture may open towards the base of the housing. Such arrangement may advantageously allow the second air flow to flow along the entire length of the aerosol generation chamber, and therefore it may improve the efficiency in purging or flushing residual aerosol droplets from the chamber.

Optionally, the bleed aperture is in fluid communication with the passage through an auxiliary passage. For example, the auxiliary passage may branch from the passage for allowing the second air flow entering the aerosol generation chamber to split from the first air flow in the passage. Such arrangement may advantageously allow said second air flow to stabilise along the auxiliary passage prior to its entrance through the bleed aperture. Alternatively, the bleed aperture may directly open to the passage without the need of an auxiliary passage.

Optionally, the auxiliary passage comprises an auxiliary flow constrictor for limiting the amount of air flow passing through the auxiliary passage. The auxiliary flow constrictor may induce a pressure drop in the second air flow thereacross, and thereby it may advantageously limit the amount of air flow entering the aerosol generation chamber through the bleed aperture.

Optionally, the auxiliary flow constrictor comprises a narrowed section of the auxiliary passage. For example, the narrowed section of the auxiliary passage may configure to have a hydraulic diameter smaller than that of the passage and therefore, it may allow a reduced amount of second air flow to flow through in comparison to that in first air flow in the passage. Advantageously, the narrowed section of the auxiliary passage may be sized to achieve a desired proportion of air flow split between passage and auxiliary passage. In some embodiments, other flow constrictors may be applied, for example an orifice, mesh and protrusions/depressions along the auxiliary passage.

Optionally, the auxiliary flow constrictor is adjustable for controlling the amount of second air flow passing through the auxiliary passage. The auxiliary flow constrictor may be adjustable mechanically or electronically. For example, the sidewall of the auxiliary passage may be deformable such that its cross sectional area may vary to control the pressure drop in the air flow thereacross. Furthermore, a part of the housing adjacent to the auxiliary passage may form from deformable material such that a user may press onto said part of housing to reduce the cross section area of the deformable auxiliary passage. Alternatively, the cross sectional area of the deformable auxiliary passage may be varied using a piezoelectric element. For example, the piezoelectric element may be energised to impart a mechanical force on the deformable auxiliary passage for varying its cross sectional area. In other embodiments, the auxiliary flow constrictor may comprise a valve positioned along the auxiliary passage or at the bleed aperture which may be actuated manually by the user or electronically through a controller of the device.

Optionally, the bleed aperture comprises a one way valve, said one way valve being configured to allow the second air flow to enter the aerosol generation chamber and to prevent leakage out of the aerosol generation chamber through the bleed aperture. For example, the one way valve may comprise a flap valve, a check valve or a duck bill valve that allows a one way flow to enter the aerosol generation chamber. Such arrangement may advantageously prevent vapour to backflow into the auxiliary passage, e.g. during vaporisation where there is an increased pressure in the aerosol generation chamber, as well as containing excess aerosol precursor and coalesced aerosol droplet collected at the base of the aerosol generation chamber.

Optionally, the bleed aperture comprises a plurality of bleed apertures distributed over a base of the aerosol generation chamber. Advantageously, such arrangement may allow the second air flow to enter the aerosol generation chamber across a broader region of the base, and thus it may lead to a more effective discharge, or flushing, of the vapour in the aerosol generation chamber. Optionally, the plurality of bleed apertures may be distributed around the periphery of the base of the aerosol generation chamber. Advantageously, such arrangement may misalign the bleed apertures with the heater, and thereby it may reduce the amount of second air flow directly impinging upon the aerosol generator.

Optionally, the smoking substitute apparatus is configured to generate an aerosol having a droplet size, dso, of at least 1 $\mu$m. Optionally, the smoking substitute apparatus is configured to generate an aerosol having a droplet size, $d_{50}$, ranged between 1 $\mu$m to 4 $\mu$m. Optionally, the smoking substitute apparatus is configured to generate an aerosol having a droplet size, $d_{50}$, ranged between 2 $\mu$m to 3 $\mu$m. Advantageously, aerosol having droplets in such size ranges may improve delivery of nicotine into the user's lung, by reducing the likelihood of nicotine deposition in the mouth and/or upper respiratory tract, e.g. in the case of oversized aerosol droplets, or not being absorbed at all, e.g. in the case of undersized aerosol droplets.

The aerosol generation chamber may be configured to discharge aerosol to the passage based on the pressure difference between the aerosol generation chamber and the passage. For example, the aerosol may be drawn into the passage due to a reduced pressure downstream, or it may be carried by the second air flow flowing through the bleed aperture, or it may be expelled from the aerosol generation chamber due to an elevated pressure resulting from vaporisation of the aerosol precursor.

The chamber outlet may be defined as an opening at the aerosol generation chamber and directly opens to a junction of the passage, e.g. the passage is adjacent to the aerosol generation chamber. Alternatively, the chamber outlet may be spaced from the passage and is in fluid communication with a junction of the passage through an aerosol channel. The aerosol channel may be configured such that pressure drop in the aerosol across the aerosol channel is negligible.

A constriction may be positioned at or adjacent to the junction. That is, the cross sectional area or hydraulic diameter of the passage at said junction may be smaller than the portion of passage immediately upstream of the junction. Such arrangement may cause the first air flow to accelerate through the constriction. According to Bernoulli's principle, as the first air flow increases in speed through the constriction, the pressure therein may reduce accordingly. This may result in a reduced pressure at the junction and therefore as it passes through the junction, the first air flow may draw the vapour and/or aerosol out of the aerosol generation chamber. Advantageously, this may induce a localised pressure drop at the junction and thereby it may allow the aerosol to be extracted from the aerosol generation chamber more effectively.

Optionally, the constriction is positioned upstream of the junction. Optionally, the constriction is positioned immediately upstream of the junction. Optionally, the constriction is spaced from the junction. The constriction may be positioned at a location upstream of the junction and configured to induce a lowered pressure at the junction for drawing out the aerosol from the aerosol generation chamber.

Optionally, the constriction comprises one or more of a venturi tube, an orifice plate and a narrowed section along the passage. For example, in the case of a venturi tube, the constriction may comprise, in the direction of air flow, a flow converging portion followed by a narrowed passage, wherein the hydraulic diameter of the narrowed passage is smaller than the flow converging portion. More specifically, the chamber outlet is configured to be in fluid communication with the narrowed passage where a localised pressure drop is created by the first air flow. Advantageously, such arrangement allows the aerosol in the aerosol generation chamber to be drawn into the narrowed passage more effectively by the localised lowered pressure.

Optionally, the passage comprises an expanded portion immediately upstream of the constriction in the passage. More specifically, the expanded portion may have a larger hydraulic diameter than the constriction. Furthermore, the expanded portion may have a larger hydraulic diameter than the passage immediate upstream thereto. Advantageously, such expanded portion may allow the first air flow to slow down before it accelerates through the constriction. As a result, it may allow a larger pressure drop across the constriction, and therefore it may lead to a more effective extraction of aerosol from the aerosol generation chamber.

Optionally, a portion of the flow passage extends alongside the aerosol generation chamber. Optionally, the aerosol generation chamber is at least partially surrounded by the passage. Optionally, the aerosol generation chamber is fully surrounded by the passage. For example, the passage may take the form of an annulus surrounding the aerosol generation chamber. Advantageously, the passage may form an effective insulation for reducing heat transfer to the external surface of the housing.

Optionally, the junction is configured to be in fluid communication with the chamber outlet through a junction inlet, and wherein said junction inlet opens towards a direction orthogonal to the first air flow at the junction. That is, the aerosol may be drawn into the junction from the side of the passage. Advantageously, this may allow the aerosol to mix with the first air flow in a more effective manner, and thereby allowing aerosol to form more uniformly.

Alternatively, the chamber outlet opens in a direction substantially parallel to the first air flow at the junction. For example, the chamber outlet may open directly to the passage. Such arrangement may allow the aerosol and the first air flow to flow concurrently, and therefore advantageously, such arrangement may reduce the turbulence when the two are mixed at the junction.

Optionally, the chamber outlet may be closed by a one way valve, such as a check valve or a duck bill value, which may advantageously prevent the first air flow from entering the aerosol generation chamber.

As the aerosol precursor is being vaporised in the aerosol generation chamber, the vaporised aerosol precursor, or the vapour, may expand in the aerosol generation chamber and thereby it may increase the internal pressure at the aerosol generation chamber. Such elevated internal pressure may advantageously help forcing the aerosol through the chamber opening into the passage, even when the user is not puffing on the mouthpiece. For example, the aerosol generation chamber may have a specified volume of air at atmospheric pressure prior to the vaporization of aerosol precursor. As the user puffs on the mouthpiece, the pressure inside the passage decreases which may cause an activation of the heater and thus the vaporisation process. At the same time, the localised low pressure region downstream of the aerosol generation chamber may draw out the higher-pressure air out of the aerosol generation chamber to equalise the pressure therein. This results in an equalized pressure across the aerosol generation chamber and the outlet.

During vaporisation, aerosol precursor may be vaporised at the heater and expand into the cavity of the aerosol generation chamber. The vaporised aerosol precursor may immediately begin to cool and some of the condensed vapour may form aerosol droplets suspended in the air. Due to vaporisation two mechanisms of pressure increase may occur in the aerosol generation chamber:

1. The vapour has a defined volume. Adding this volume of gaseous vapour to the defined volume of air inside the aerosol generation chamber may increase the pressure therein.
2. The aerosol droplets has a specified volume. These liquid droplets may displace the same volume of air.

Both effects may increase the pressure with respect to the lower pressure in the first air flow. This may aid the expulsion of vapour and aerosol from the aerosol generation chamber during vaporisation.

By reducing or limiting the volume of the aerosol generation chamber, the expulsion of the aerosol from the chamber may be made more efficient. That is, a smaller aerosol generation chamber volume may result in greater pressure rise in the aerosol generation chamber for a given vapor generation rate. More specifically, increasing the internal volume of the aerosol generation chamber may reduce the amount of vapour and/or aerosol that is able to be ejected from the aerosol generation chamber during a puff, with the aerosol generated with an increase average droplet size. On the other hand, reducing the internal volume of the aerosol generation chamber increases the amount of vapour and/or aerosol during a puff with a reduction in the average aerosol droplet size. Optionally, the aerosol generation chamber is configured to have an internal volume ranging between $68mm^3$ to $680mm^3$. Optionally, the length of the aerosol generation chamber ranges between 2mm to 20mm. Such arrangements may advantageously allow the aerosol to be expulsed from the aerosol generation chamber at a rate greater than 0.1 mg/second during a puff.

The aerosol generation chamber may open towards the second end of the housing, or the mouthpiece. More specifically, an open end of the aerosol generation chamber may direct towards an upward direction during use. Advantageously, this may help containing any excess aerosol precursor and coalesced aerosol droplets formed in the aerosol generation chamber.

Optionally, the smoking substitute apparatus further comprises a tank for storing a reservoir of liquid aerosol precursor, the tank is spaced from the aerosol generation chamber. In the case where the aerosol generator is a heater, the tank is arranged to be in fluid communication with a wick of the heater through a liquid conduit. The tank may be provided at a location between the aerosol generation chamber and the mouthpiece, and therefore the wick may not extend directly from the aerosol generation chamber into the tank. Advantageously, this may allow the tank to be positioned flexibly at various positions within the housing, and thereby such arrangement may free up the space required for the constriction.

The liquid conduit may be sized to control the feed rate of the aerosol precursor from the tank to the wick. Optionally, the liquid conduit has a hydraulic diameter ranging from 0.01mm to 10 mm for controlling the flow rate of the aerosol precursor from the tank towards the wick. Optionally, the liquid conduit has a hydraulic diameter ranging from 0.01mm to 5 mm. Optionally, the liquid conduit has a hydraulic diameter of at least 0.1mm. Optionally, the liquid conduit has a hydraulic diameter of up to 1 mm. Advantageously, such arrangement may limit the flow of aerosol precursor towards the wick under gravity, and thereby it may reduce the leakage of excess aerosol precursor into the aerosol generation chamber.

Furthermore, the inclusion of a liquid conduit may solve the problem of insufficient supply of aerosol precursor at the wick. For example, by having a reduced amount of air flow in the aerosol generation chamber, the associated driving force, e.g. the pressure drop in the aerosol generation chamber, for drawing out aerosol precursor from the tank may reduce accordingly. By relocating the tank to a position between the aerosol generation chamber and the outlet, e.g. the tank is located above the aerosol generation chamber in an upright orientation during use, the aerosol precursor may flow from the tank towards the wick under additional hydraulic head and thereby mitigates such problem. Advantageously, by providing a liquid conduit that is configured to allow sufficient flow of aerosol precursor, the wick may be prevented from drying out during vaporisation.

In addition, different consumable having liquid conduits with different hydraulic diameters may be used to accommodate different types of users. For example, consumables having liquid conduits with broader hydraulic diameter may be catered for heavy users taking longer puffs, where an increased flow of aerosol precursor may reduce the likelihood of drying out at the wick. Consumables having liquid conduits with narrower hydraulic diameter may be catered for light users who take shorter puffs, where a reduced flow of aerosol precursor may reduce the amount of excess aerosol precursor at the wick.

Optionally, the aerosol generator is located at a position immediately upstream of the chamber outlet. More specifically, the aerosol generator may be positioned in the aerosol generation chamber by the chamber outlet. Advantageously, this may shorten the path of travel for the aerosol and thereby allow the aerosol to be drawn into the passage more effectively.

Alternatively, the aerosol generator is spaced from the chamber outlet. Advantageously, such arrangement may reduce the likelihood of first the air flow entering into the aerosol generation chamber through the chamber outlet, and thereby limiting the turbulence in the vicinity of the aerosol generator. Further, this may increase the residence time of the vapour in the aerosol generation chamber and thus it may allow some aerosol droplets to form and even coalesce before being entrained in the first air flow. Optionally, the aerosol generator is separated from the chamber outlet at a distance of at least 10 mm. optionally, the aerosol generator is separated from the chamber outlet at a distance ranged from 10mm to 50mm.

Optionally, the aerosol generation chamber is configured to have a uniform cross sectional profile along its length. Optionally, the chamber outlet is configured to have the same cross sectional profile as the aerosol generation chamber. For example, the aerosol flow path along the length of the aerosol generation chamber may have the same cross-sectional area. Advantageously, this may reduce turbulence, as well as fluctuation in pressure in the aerosol flow path and thereby such arrangement may lead to an increase in the size of aerosol droplets.

The smoking substitute apparatus may be in the form of a consumable. The consumable may be configured for engagement with a main body. When the consumable is engaged with the main body, the combination of the consumable and the main body may form a smoking substitute system such as a closed smoking substitute system. For example, the consumable may comprise components of the system that are disposable, and the main body may comprise non-disposable or non-consumable components (e.g. power supply, controller, sensor, etc.) that facilitate the generation and/or delivery of aerosol by the consumable. In such an embodiment, the aerosol precursor (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol precursor (e.g. e-liquid) of the system may be replenished by re-filling, e.g. a reservoir of the smoking substitute apparatus, with the aerosol precursor (rather than replacing a consumable component of the apparatus).

In light of this, it should be appreciated that some of the features described herein as being part of the smoking substitute apparatus may alternatively form part of a main body for engagement with the smoking substitute apparatus. This may be the case in particular when the smoking substitute apparatus is in the form of a consumable.

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting, or the like.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a reservoir configured to store an aerosol precursor, such as an e-liquid. The e-liquid may, for example, comprise a base liquid. The e-liquid may further comprise nicotine. The base liquid may include propylene glycol and/or vegetable glycerine. The e-liquid may be substantially flavourless. That is, the e-liquid may not contain any deliberately added additional flavourant and may consist solely of a base liquid of propylene glycol and/or vegetable glycerine and nicotine.

The reservoir may be in the form of a tank. At least a portion of the tank may be light-transmissive. For example, the tank may comprise a window to allow a user to visually assess the quantity of e-liquid in the tank. A housing of the smoking substitute apparatus may comprise a corresponding aperture (or slot) or window that may be aligned with a light-transmissive portion (e.g. window) of the tank. The reservoir may be referred to as a "clearomizer" if it includes a window, or a "cartomizer" if it does not.

The passage may be at least partially defined by the tank. The tank may substantially (or fully) define the passage, for at least a part of the length of the passage. In this respect, the tank may surround the passage, e.g. in an annular arrangement around the passage.

The aerosol generator may comprise a wick. The aerosol generator may further comprise a heater. The wick may comprise a porous material, capable of wicking the aerosol precursor. A portion of the wick may be exposed to air flow in the passage. The wick may also comprise one or more portions in contact with liquid stored in the reservoir. For example, opposing ends of the wick may protrude into the reservoir and an intermediate portion (between the ends) may extend across the passage so as to be exposed to air flow in the passage. Thus, liquid may be drawn (e.g. by capillary action) along the wick, from the reservoir to the portion of the wick exposed to air flow.

The heater may comprise a heating element, which may be in the form of a filament wound about the wick (e.g. the filament may extend helically about the wick in a coil configuration). The heating element may be wound about the intermediate portion of the wick that is exposed to air. The heating element may be electrically connected (or connectable) to a power source. Thus, in operation, the power source may apply a voltage across the heating element so as to heat the heating element by resistive heating. This may cause liquid stored in the wick (i.e. drawn from the tank) to be heated so as to form a vapour and become entrained in air flowing through the passage. This vapour may subsequently cool to form an aerosol in the passage, typically downstream from the heating element.

In use, the user may puff on a mouthpiece of the smoking substitute apparatus, i.e. draw on the smoking substitute apparatus by inhaling, to draw in an air stream therethrough. A minority portion, of the air stream (also referred to as a "second air flow") may pass through the aerosol generation chamber so as to entrain the vapour generated at the heater. That is, such a second air flow may be heated by the heater (although typically only to a limited extent) as it passes through the aerosol generation chamber. A portion of the air stream (also referred to as a "first air flow" or "bypass air flow") bypasses the aerosol generation chamber is directed to mix with the generated aerosol downstream from the aerosol generation chamber. That is, the first air flow may be an air stream at an ambient temperature and may not be directly heated at all by the heater. The first air flow may combine with the second air flow for diluting the aerosol contained therein. The first air flow may merge with the second air flow along the passage downstream from the aerosol generation chamber.

As a user puffs on the mouthpiece, vaporised e-liquid entrained in the passing air flow may be drawn towards the outlet of passage. The vapour may cool, and thereby nucleate and/or condense along the passage to form a plurality of aerosol droplets, e.g. nicotine-containing aerosol droplets. A portion of these aerosol droplets may be delivered to and be absorbed at a target delivery site, e.g. a user's lung, whilst a portion of the aerosol droplets may instead adhere onto other parts of the user's respiratory tract, e.g. the user's oral cavity and/or throat. Typically, in some known smoking substitute apparatuses, the aerosol droplets as measured at the outlet of the passage, e.g. at the mouthpiece, may have a droplet size, dso, of less than 1$\mu$m.

The particle droplet size, $d_{50}$, of an aerosol may be measured by a laser diffraction technique. For example, the stream of aerosol output from the outlet of the passage may be drawn through a Malvern Spraytec laser diffraction system, where the intensity and pattern of scattered laser light are analysed to calculate the size and size distribution of aerosol droplets. As will be readily understood, the particle size distribution may be expressed in terms of dio, $d_{50}$ and $d_{90}$, for example. Considering a cumulative plot of the volume of the particles measured by the laser diffraction technique, the $d_{10}$ particle size is the particle size below which 10% by volume of the sample lies. The $d_{50}$ particle size is the particle size below which 50% by volume of the sample lies. The $d_{90}$ particle size is the particle size below which 90% by volume of the sample lies. Unless otherwise indicated herein, the particle size measurements are volume-based particle size measurements, rather than number-based or mass-based particle size measurements.

The smoking substitute apparatus (or main body engaged with the smoking substitute apparatus) may comprise a power source. The power source may be electrically connected (or connectable) to a heater of the smoking substitute apparatus (e.g. when the smoking substitute apparatus is engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface of the main body may be configured to transfer electrical power from the power source to a heater of the consumable via the electrical interface of the consumable.

The electrical interface of the smoking substitute apparatus may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an identification means, which may, for example, be in the form of an RFID reader, a barcode or QR code reader. This identification means may be able to identify a characteristic (e.g. a type) of a consumable engaged with the main body. In this respect, the consumable may include any one or more of an RFID chip, a barcode or QR code, or memory within which is an identifier and which can be interrogated via the identification means.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may be provided in the consumable or alternatively may be provided in the main body.

The term "flavourant" is used to describe a compound or combination of compounds that provide flavour and/or aroma. For example, the flavourant may be configured to interact with a sensory receptor of a user (such as an olfactory or taste receptor). The flavourant may include one or more volatile substances.

The flavourant may be provided in solid or liquid form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

According to a second aspect there is provided a smoking substitute system for generating an aerosol, comprising:

i) the smoking substitute apparatus of the first aspect; and

ii) a main body configured to engage with the smoking substitute apparatus; wherein the main body comprises a controller and a power source configured to energise the heater.

According to third aspect there is provided a method of using the smoking substitute apparatus of the first aspect, comprising:

i) generating the aerosol with the aerosol generator; and

ii) drawing on the apparatus to cause air to enter the aerosol generation chamber in an amount that contributes not more than 35% of the air flow at the outlet.

The present inventors consider that a flow rate of 1.3 L min⁻¹ is towards the lower end of a typical user expectation of flow rate through a conventional cigarette and therefore through a user-acceptable smoking substitute apparatus. The present inventors further consider that a flow rate of 2.0 L min⁻¹ is towards the higher end of a typical user expectation of flow rate through a conventional cigarette and therefore through a user-acceptable smoking substitute apparatus. Embodiments of the present invention therefore provide an aerosol with advantageous particle size characteristics across a range of flow rates of air through the apparatus.

The aerosol may have a Dv50 of at least 1.1 $\mu$m, at least 1.2 $\mu$m, at least 1.3 $\mu$m, at least 1.4 $\mu$m, at least 1.5 $\mu$m, at least 1.6 $\mu$m, at least 1.7 $\mu$m, at least 1.8 $\mu$m, at least 1.9 $\mu$m or at least 2.0 $\mu$m.

The aerosol may have a Dv50 of not more than 4.9 $\mu$m, not more than 4.8 $\mu$m, not more than 4.7 $\mu$m, not more than 4.6 $\mu$m, not more than 4.5 $\mu$m, not more than 4.4 $\mu$m, not more than 4.3 $\mu$m, not more than 4.2 $\mu$m, not more than 4.1 $\mu$m, not more than 4.0 $\mu$m, not more than 3.9 $\mu$m, not more than 3.8 $\mu$m, not more than 3.7 $\mu$m, not more than 3.6 $\mu$m, not more than 3.5 $\mu$m, not more than 3.4 $\mu$m, not more than 3.3 $\mu$m, not more than 3.2 $\mu$m, not more than 3.1 $\mu$m or not more than 3.0 $\mu$m.

A particularly preferred range for Dv50 of the aerosol is in the range 2-3 $\mu$m.

The air inlet, flow passage, outlet and the vaporisation chamber may be configured so that, when the air flow rate inhaled by the user through the apparatus is 1.3 L min⁻¹, the average magnitude of velocity of air in the vaporisation chamber is in the range 0-1.3 ms⁻¹. The average magnitude velocity of air may be calculated based on knowledge of the geometry of the vaporisation chamber and the flow rate.

When the air flow rate inhaled by the user through the apparatus is 1.3 L min⁻¹, the average magnitude of velocity of air in the vaporisation chamber may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min⁻¹, the average magnitude of velocity of air in the vaporisation chamber may be at most 1.2 ms⁻¹, at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

The air inlet, flow passage, outlet and the vaporisation chamber may be configured so that, when the air flow rate inhaled by the user through the apparatus is 2.0 L min⁻¹, the average magnitude of velocity of air in the vaporisation chamber is in the range 0-1.3 ms⁻¹. The average magnitude velocity of air may be calculated based on knowledge of the geometry of the vaporisation chamber and the flow rate.

When the air flow rate inhaled by the user through the apparatus is 2.0 L min⁻¹, the average magnitude of velocity of air in the vaporisation chamber may be at least 0.001 ms⁻¹, or at least 0.005 ms⁻¹, or at least 0.01 ms⁻¹, or at least 0.05 ms⁻¹.

When the air flow rate inhaled by the user through the apparatus is 2.0 L min⁻¹, the average magnitude of velocity of air in the vaporisation chamber may be at most 1.2 ms⁻¹, at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

When the calculated average magnitude of velocity of air in the vaporisation chamber is in the ranges specified, it is considered that the resultant aerosol particle size is advantageously controlled to be in a desirable range. It is further considered that the configuration of the apparatus can be selected so that the average magnitude of velocity of air in the vaporisation chamber can be brought within the ranges specified, at the exemplary flow rate of 1.3 L min⁻¹ and/or the exemplary flow rate of 2.0 L min⁻¹.

The aerosol generator may comprise a vaporiser element loaded with aerosol precursor, the vaporiser element being heatable by a heater and presenting a vaporiser element surface to air in the vaporisation chamber. A vaporiser element region may be defined as a volume extending outwardly from the vaporiser element surface to a distance of 1 mm from the vaporiser element surface.

The air inlet, flow passage, outlet and the vaporisation chamber may be configured so that, when the air flow rate inhaled by the user through the apparatus is 1.3 L min⁻¹, the average magnitude of velocity of air in the vaporiser element region is in the range 0-1.2 ms⁻¹. The average magnitude of velocity of air in the vaporiser element region may be calculated using computational fluid dynamics.

When the air flow rate inhaled by the user through the apparatus is 1.3 L min⁻¹, the average magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min$^{-1}$, the average magnitude of velocity of air in the vaporiser element region may be at most 1.1 ms$^{-1}$, at most 1.0 ms$^{-1}$, at most 0.9 ms$^{-1}$, at most 0.8 ms$^{-1}$, at most 0.7 ms$^{-1}$ or at most 0.6 ms$^{-1}$.

The air inlet, flow passage, outlet and the vaporisation chamber may be configured so that, when the air flow rate inhaled by the user through the apparatus is 2.0 L min$^{-1}$, the average magnitude of velocity of air in the vaporiser element region is in the range 0-1.2 ms$^{-1}$. The average magnitude of velocity of air in the vaporiser element region may be calculated using computational fluid dynamics.

When the air flow rate inhaled by the user through the apparatus is 2.0 L min$^{-1}$, the average magnitude of velocity of air in the vaporiser element region may be at least 0.001 ms$^{-1}$, or at least 0.005 ms$^{-1}$, or at least 0.01 ms$^{-1}$, or at least 0.05 ms$^{-1}$.

When the air flow rate inhaled by the user through the apparatus is 2.0 L min$^{-1}$, the average magnitude of velocity of air in the vaporiser element region may be at most 1.1 ms$^{-1}$, at most 1.0 ms$^{-1}$, at most 0.9 ms$^{-1}$, at most 0.8 ms$^{-1}$, at most 0.7 ms$^{-1}$ or at most 0.6 ms$^{-1}$.

When the average magnitude of velocity of air in the vaporiser element region is in the ranges specified, it is considered that the resultant aerosol particle size is advantageously controlled to be in a desirable range. It is further considered that the velocity of air in the vaporiser element region is more relevant to the resultant particle size characteristics than consideration of the velocity in the vaporisation chamber as a whole. This is in view of the significant effect of the velocity of air in the vaporiser element region on the cooling of the vapour emitted from the vaporiser element surface.

Additionally or alternatively is it relevant to consider the maximum magnitude of velocity of air in the vaporiser element region.

Therefore, the air inlet, flow passage, outlet and the vaporisation chamber may be configured so that, when the air flow rate inhaled by the user through the apparatus is 1.3 L min$^{-1}$, the maximum magnitude of velocity of air in the vaporiser element region is in the range 0-2.0 ms$^{-1}$.

When the air flow rate inhaled by the user through the apparatus is 1.3 L min$^{-1}$, the maximum magnitude of velocity of air in the vaporiser element region may be at least 0.001 ms$^{-1}$, or at least 0.005 ms$^{-1}$, or at least 0.01 ms$^{-1}$, or at least 0.05 ms$^{-1}$.

When the air flow rate inhaled by the user through the apparatus is 1.3 L min$^{-1}$, the maximum magnitude of velocity of air in the vaporiser element region may be at most 1.9 ms$^{-1}$, at most 1.8 ms$^{-1}$, at most 1.7 ms$^{-1}$, at most 1.6 ms$^{-1}$, at most 1.5 ms$^{-1}$, at most 1.4 ms$^{-1}$, at most 1.3 ms$^{-1}$ or at most 1.2 ms$^{-1}$.

The air inlet, flow passage, outlet and the vaporisation chamber may be configured so that, when the air flow rate inhaled by the user through the apparatus is 2.0 L min$^{-1}$, the maximum magnitude of velocity of air in the vaporiser element region is in the range 0-2.0 ms$^{-1}$.

When the air flow rate inhaled by the user through the apparatus is 2.0 L min$^{-1}$, the maximum magnitude of velocity of air in the vaporiser element region may be at least 0.001 ms$^{-1}$, or at least 0.005 ms$^{-1}$, or at least 0.01 ms$^{-1}$, or at least 0.05 ms$^{-1}$.

When the air flow rate inhaled by the user through the apparatus is 2.0 L min$^{-1}$, the maximum magnitude of velocity of air in the vaporiser element region may be at most 1.9 ms$^{-1}$, at most 1.8 ms$^{-1}$, at most 1.7 ms$^{-1}$, at most 1.6 ms$^{-1}$, at most 1.5 ms$^{-1}$, at most 1.4 ms$^{-1}$, at most 1.3 ms$^{-1}$ or at most 1.2 ms$^{-1}$.

It is considered that configuring the apparatus in a manner to permit such control of velocity of the airflow at the vaporiser permits the generation of aerosols with particularly advantageous particle size characteristics, including Dv50 values.

Additionally or alternatively is it relevant to consider the turbulence intensity in the vaporiser chamber in view of the effect of turbulence on the particle size of the generated aerosol. For example, the air inlet, flow passage, outlet and the vaporisation chamber may be configured so that, when the air flow rate inhaled by the user through the apparatus is 1.3 L min$^{-1}$, the turbulence intensity in the vaporiser element region is not more than 1%.

When the air flow rate inhaled by the user through the apparatus is 1.3 L min$^{-1}$, the turbulence intensity in the vaporiser element region may be not more than 0.95%, not more than 0.9%, not more than 0.85%, not more than 0.8%, not more than 0.75%, not more than 0.7%, not more than 0.65% or not more than 0.6%.

It is considered that configuring the apparatus in a manner to permit such control of the turbulence intensity in the vaporiser element region permits the generation of aerosols with particularly advantageous particle size characteristics, including Dv50 values.

Following detailed investigations, the inventors consider, without wishing to be bound by theory, that the particle size characteristics of the generated aerosol may be determined by the cooling rate experienced by the vapour after emission from the vaporiser element (e.g. wick). In particular, it appears that imposing a relatively slow cooling rate on the vapour has the effect of generating aerosols with a relatively large particle size. The parameters discussed above (velocity and turbulence intensity) are considered to be mechanisms for implementing a particular cooling dynamic to the vapour.

More generally, it is considered that the air inlet, flow passage, outlet and the vaporisation chamber may be configured so that a desired cooling rate is imposed on the vapour. The particular cooling rate to be used depends of course on the nature of the aerosol precursor and other conditions. However, for a particular aerosol precursor it is possible to define a set of testing conditions in order to define the cooling rate, and by extension this imposes limitations on the configuration of the apparatus to permit such cooling rates as are shown to result in advantageous aerosols. Accordingly, the air inlet, flow passage, outlet and the vaporisation chamber may be configured so that the cooling rate of the vapour is such that the time taken to cool to 50 °C is not less than 16 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 1.3 L min$^{-1}$.

Additionally or alternatively, the air inlet, flow passage, outlet and the vaporisation chamber may be configured so that the cooling rate of the vapour is such that the time taken to cool to 50 °C is not less than 16 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 2.0 L min$^{-1}$.

Cooling of the vapour such that the time taken to cool to 50 °C is not less than 16 ms corresponds to an equivalent linear cooling rate of not more than 10 °C/ms.

The equivalent linear cooling rate of the vapour to 50 °C may be not more than 9 °C/ms, not more than 8 °C/ms, not more than 7 °C/ms, not more than 6 °C/ms or not more than 5 °C/ms.

Cooling of the vapour such that the time taken to cool to 50 °C is not less than 32 ms corresponds to an equivalent linear cooling rate of not more than 5 °C/ms.

The testing protocol set out above considers the cooling of the vapour (and subsequent aerosol) to a temperature of 50 °C. This is a temperature which can be considered to be suitable for an aerosol to exit the apparatus for inhalation by a user without causing significant discomfort. It is also possible to consider cooling of the vapour (and subsequent aerosol) to a temperature of 75 °C. Although this temperature is possibly too high for comfortable inhalation, it is considered that the particle size characteristics of the aerosol are substantially settled by the time the aerosol cools to this temperature (and they may be settled at still higher temperature).

Accordingly, the air inlet, flow passage, outlet and the vaporisation chamber may be configured so that the cooling rate of the vapour is such that the time taken to cool to 75 °C is not less than 4.5 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 1.3 L min$^{-1}$.

Additionally or alternatively, the air inlet, flow passage, outlet and the vaporisation chamber may be configured so that the cooling rate of the vapour is such that the time taken to cool to 75 °C is not less than 4.5 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 2.0 L min$^{-1}$.

Cooling of the vapour such that the time taken to cool to 75 °C is not less than 4.5 ms corresponds to an equivalent linear cooling rate of not more than 30 °C/ms.

The equivalent linear cooling rate of the vapour to 75 °C may be not more than 29 °C/ms, not more than 28 °C/ms, not more than 27 °C/ms, not more than 26 °C/ms, not more than 25 °C/ms, not more than 24 °C/ms, not more than 23 °C/ms, not more than 22 °C/ms, not more than 21 °C/ms, not more than 20 °C/ms, not more than 19 °C/ms, not more than 18 °C/ms, not more than 17 °C/ms, not more than 16 °C/ms, not more than 15 °C/ms, not more than 14 °C/ms, not more than 13 °C/ms, not more than 12 °C/ms, not more than 11 °C/ms or not more than 10 °C/ms.

Cooling of the vapour such that the time taken to cool to 75 °C is not less than 13 ms corresponds to an equivalent linear cooling rate of not more than 10 °C/ms.

It is considered that configuring the apparatus in a manner to permit such control of the cooling rate of the vapour permits the generation of aerosols with particularly advantageous particle size characteristics, including Dv50 values.

The invention includes the combination of the aspects and preferred features described except where such a combination is clearly impermissible or expressly avoided.

## *Summary of the Figures*

So that the invention may be understood, and so that further aspects and features thereof may be appreciated, embodiments illustrating the principles of the invention will now be discussed in further detail with reference to the accompanying figures, in which:

Figure 1 illustrates a set of rectangular tubes for use in experiments to assess the effect of flow and cooling conditions at the wick on aerosol properties. Each tube has the same depth and length but different width.

Figure 2 shows a schematic perspective longitudinal cross sectional view of an example rectangular tube with a wick and heater coil installed.

Figure 3 shows a schematic transverse cross sectional view an example rectangular tube with a wick and heater coil installed. In this example, the internal width of the tube is 12 mm.

Figures 4A-4D show air flow streamlines in the four devices used in a turbulence study.

Figure 5 shows the experimental set up to investigate the influence of inflow air temperature on aerosol particle size, in order to investigate the effect of vapour cooling rate on aerosol generation.

Figure 6 shows a schematic longitudinal cross sectional view of a first smoking substitute apparatus (pod 1) used to assess influence of inflow air temperature on aerosol particle size.

Figure 7 shows a schematic longitudinal cross sectional view of a second smoking substitute apparatus (pod 2) used to assess influence of inflow air temperature on aerosol particle size.

Figure 8A shows a schematic longitudinal cross sectional view of a third smoking substitute apparatus (pod 3) used to assess influence of inflow air temperature on aerosol particle size. Figure 8B shows a schematic longitudinal cross sectional view of the same third smoking substitute apparatus (pod 3) in a direction orthogonal to the view taken in Figure 8A.

Figure 9 shows a plot of aerosol particle size (Dv50) experimental results against calculated air velocity.

Figure 10 shows a plot of aerosol particle size (Dv50) experimental results against the flow rate through the apparatus for a calculated air velocity of 1 m/s.

Figure 11 shows a plot of aerosol particle size (Dv50) experimental results against the average magnitude of the velocity in the vaporiser surface region, as obtained from CFD modelling.

Figure 12 shows a plot of aerosol particle size (Dv50) experimental results against the maximum magnitude of the velocity in the vaporiser surface region, as obtained from CFD modelling.

Figure 13 shows a plot of aerosol particle size (Dv50) experimental results against the turbulence intensity.

Figure 14 shows a plot of aerosol particle size (Dv50) experimental results dependent on the temperature of the air and the heating state of the apparatus.

Figure 15 shows a plot of aerosol particle size (Dv50) experimental results against vapour cooling rate to 50°C.

Figure 16 shows a plot of aerosol particle size (Dv50) experimental results against vapour cooling rate to 75°C.

Figure 17 is a schematic front view of a smoking substitute system, according to a first reference arrangement, in an engaged position;

Figure 18 is a schematic front view of the smoking substitute system of the first reference arrangement in a disengaged position;

Figure 19 is a schematic longitudinal cross sectional view of a smoking substitute apparatus of the first reference arrangement; and

Figure 20 is an enlarged schematic cross sectional view of part of the air passage and aerosol generation chamber of the first reference arrangement;

Figure 21A is a schematic longitudinal cross sectional view of a smoking substitute apparatus of the first embodiment;

Figure 21B is an enlarged schematic cross sectional view of a junction of the first embodiment as shown in Figure 21A;

Figure 21C is an enlarged schematic cross sectional view of a base of the first embodiment as shown in

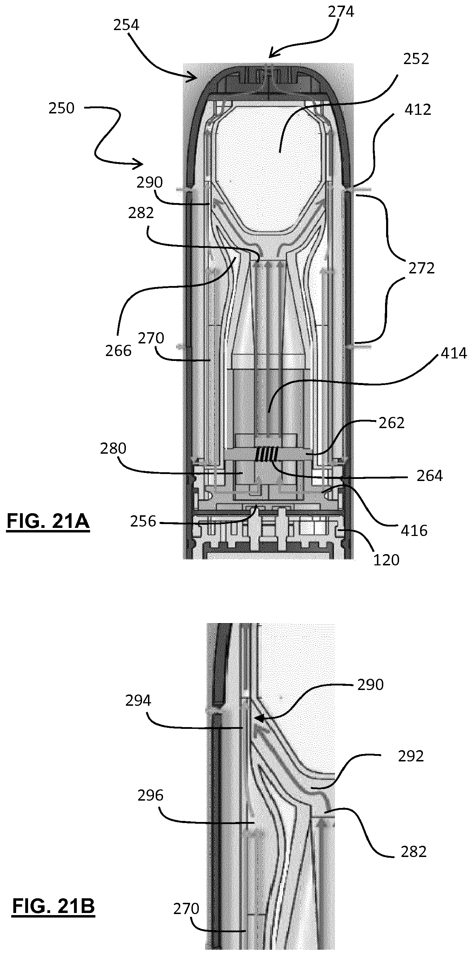

Figure 21A; and

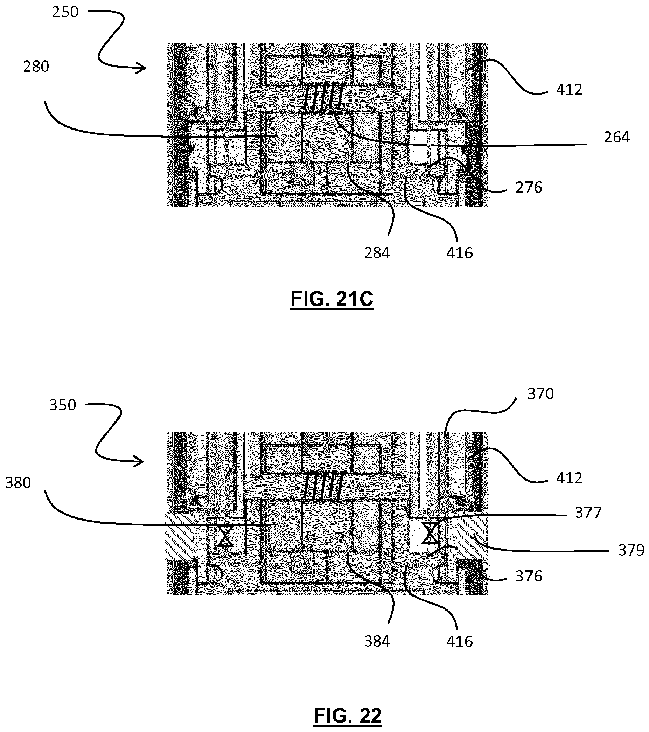

Figure 22 is an enlarged schematic cross sectional view of a base of a smoking substitute apparatus of the second embodiment.

## Detailed Description of the Invention

Further background to the present invention and further aspects and embodiments of the present invention will now be discussed with reference to the accompanying figures. Further aspects and embodiments will be apparent to those skilled in the art. The contents of all documents mentioned in this text are incorporated herein by reference in their entirety.

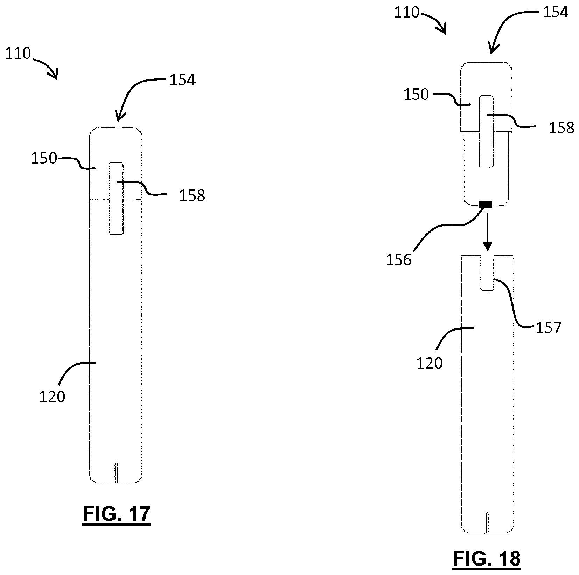

Figures 17 and 18 illustrate a smoking substitute system in the form of an e-cigarette system 110. The system 110 comprises a main body 120 of the system 110, and a smoking substitute apparatus in the form of an e-cigarette consumable (or "pod") 150. In the illustrated embodiment the consumable 150 (sometimes referred to herein as a smoking substitute apparatus) is removable from the main body 120, so as to be a replaceable component of the system 110. The e-cigarette system 110 is a closed system in the sense that it is not intended that the consumable should be refillable with e-liquid by a user.

As is apparent from Figures 17 and 18, the consumable 150 is configured to engage the main body 120. Figure 17 shows the main body 120 and the consumable 150 in an engaged state, whilst Figure 18 shows the main body 120 and the consumable 150 in a disengaged state. When engaged, a portion of the consumable 150 is received in a cavity of corresponding shape in the main body 120 and is retained in the engaged position by way of a snap-engagement mechanism. In other embodiments, the main body 120 and consumable 150 may be engaged by screwing one into (or onto) the other, or through a bayonet fitting, or by way of an interference fit.

The system 110 is configured to vaporise an aerosol precursor, which in the illustrated embodiment is in the form of a nicotine-based e-liquid 160. The e-liquid 160 comprises nicotine and a base liquid including propylene glycol and/or vegetable glycerine. In the present embodiment, the e-liquid 160 is flavoured by a flavourant. In other embodiments, the e-liquid 160 may be flavourless and thus may not include any added flavourant.

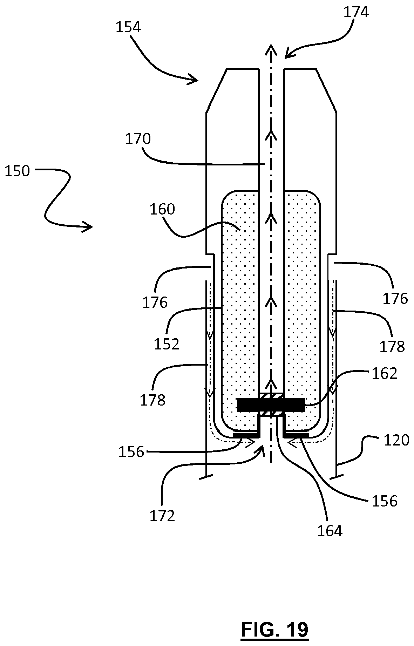

Figure 19 shows a schematic longitudinal cross sectional view of a smoking substitute apparatus according to a reference arrangement that is configured to form part of the smoking substitute system shown in Figures 17 and 18. The smoking substitute apparatus, or consumable 150 as shown in Figure 19 is provided as a reference arrangement to illustrate the features of a consumable 150 and its interaction with the main body 120. In Figure 19, the e-liquid 160 is stored within a reservoir in the form of a tank 152 that forms part of the consumable 150. In the illustrated embodiment, the consumable 150 is a "single-use" consumable 150. That is, upon exhausting the e-liquid 160 in the tank 152, the intention is that the user disposes of the entire consumable 150. The term "single-use" does not necessarily mean the consumable is designed to be disposed of after a single smoking session. Rather, it defines the consumable 150 is not arranged to be refilled after the e-liquid contained in the tank 152 is depleted. The tank may include a vent (not shown) to allow ingress of air to replace e-liquid that has been used from the tank. The consumable 150 preferably includes a window 158 (see Figures 17 and 18), so that the amount of e-liquid in the tank 152 can be visually assessed. The main body 120 includes a slot 157 so that the window 158 of the consumable 150 can be seen whilst the rest of the tank 152 is obscured from view when the consumable 150 is received in the cavity of the main body 120. The consumable 150 may be referred to as a "clearomizer" when it includes a window 158, or a "cartomizer" when it does not.

In other embodiments, the e-liquid (i.e. aerosol precursor) may be the only part of the system that is truly "single-use". That is, the tank may be refillable with e-liquid or the e-liquid may be stored in a non-consumable component of the system. For example, in such other embodiments, the e-liquid may be stored in a tank located in the main body or stored in another component that is itself not single-use (e.g. a refillable cartomizer).

The external wall of tank 152 is provided by a casing of the consumable 150. The tank 152 annularly surrounds, and thus defines a portion of, a passage 170 that extends between a vaporiser inlet 172 and an outlet 174 at opposing ends of the consumable 150. In this respect, the passage 170 comprises an upstream end at the end of the consumable 150 that engages with the main body 120, and a downstream end at an opposing end of the consumable 150 that comprises a mouthpiece 154 of the system 110.

When the consumable 150 is received in the cavity of the main body 120 as shown in Figure 19, a plurality of device air inlets 176 are formed at the boundary between the casing of the consumable and the casing of the main body. The device air inlets 176 are in fluid communication with the vaporiser inlet 172 through an inlet flow channel 178 formed in the cavity of the main body which is of corresponding shape to receive a part of the consumable 150. Air from outside of the system 110 can therefore be drawn into the passage 170 through the device air inlets 176 and the inlet flow channels 178.

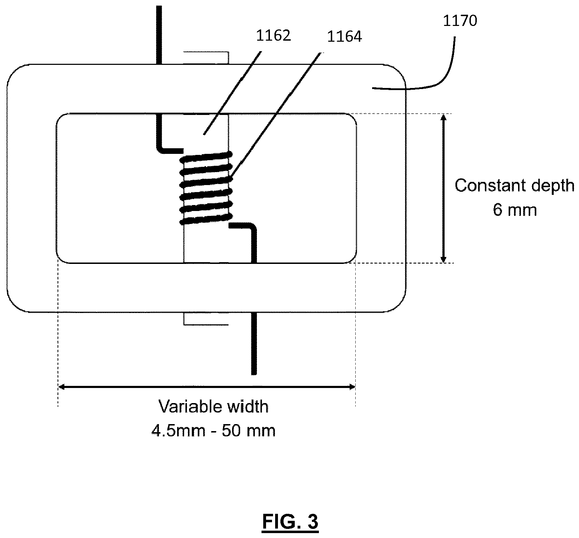

When the consumable 150 is engaged with the main body 120, a user can inhale (i.e. take a puff) via the mouthpiece 154 so as to draw air through the passage 170, and so as to form an air flow (indicated by the dashed arrows in Figure 3) in a direction from the vaporiser inlet 172 to the outlet 174. Although not illustrated, the passage 170 may be partially defined by a tube (e.g. a metal tube) extending through the consumable 150. In Figure 19, for simplicity, the passage 170 is shown with a substantially circular cross-sectional profile with a constant diameter along its length. In other arrangements and in some embodiments, the passage may have other cross-sectional profiles, such as oval shaped or polygonal shaped profiles. Further, in other arrangements and some embodiments, the cross sectional profile and the diameter (or hydraulic diameter) of the passage may vary along its longitudinal axis.

The smoking substitute system 110 is configured to vaporise the e-liquid 160 for inhalation by a user. To provide this operability, the consumable 150 comprises an aerosol generator such as a heater, the heater having a porous wick 162 and a resistive heating element in the form of a heating filament 164 that is helically wound (in the form of a coil) around a portion of the porous wick 162. The porous wick 162 extends across the passage 170 (i.e. transverse to a longitudinal axis of the passage 170 and thus also transverse to the air flow along the passage 170 during use) and opposing ends of the wick 162 extend into the tank 152 (so as to be immersed in the e-liquid 160). In this way, e-liquid 160 contained in the tank 152 is conveyed from the opposing ends of the porous wick 162 to a central portion of the porous wick 162 so as to be exposed to the air flow in the passage 170.

The helical filament 164 is wound about the exposed central portion of the porous wick 162 and is electrically connected to an electrical interface in the form of electrical contacts 156 mounted at the end of the consumable that is proximate the main body 120 (when the consumable and the main body are engaged). When the consumable 150 is engaged with the main body 120, electrical contacts 156 make contact with corresponding electrical contacts (not shown) of the main body 120. The main body electrical contacts are electrically connectable to a power source (not shown) of the main body 120, such that (in the engaged position) the filament 164 is electrically connectable to the power source. In this way, power can be supplied by the main body 120 to the filament 164 in order to heat the filament 164. This heats the porous wick 162 which causes e-liquid 160 conveyed by the porous wick 162 to vaporise and thus to be released from the porous wick 162. The vaporised e-liquid becomes entrained in the air flow and, as it cools in the air flow (between the heated wick and the outlet 174 of the passage 170), condenses to form an aerosol. This aerosol is then inhaled, via the mouthpiece 154, by a user of the system 110. As e-liquid is lost from the heated portion of the wick, further e-liquid is drawn along the wick from the tank to replace the e-liquid lost from the heated portion of the wick.

The filament 164 and the exposed central portion of the porous wick 162 are positioned across the passage 170. More specifically, the part of passage that contains the filament 164 and the exposed portion of the porous wick 162 forms an aerosol generation chamber. In the illustrated example, the aerosol generation chamber has the same cross-sectional diameter as the passage 170. However, in other embodiments the aerosol generation chamber may have a different cross sectional profile as the passage 170. For example, the aerosol generation chamber may have a larger cross sectional diameter than at least some of the downstream part of the passage 170 so as to enable a longer residence time for the air inside the aerosol generation chamber.

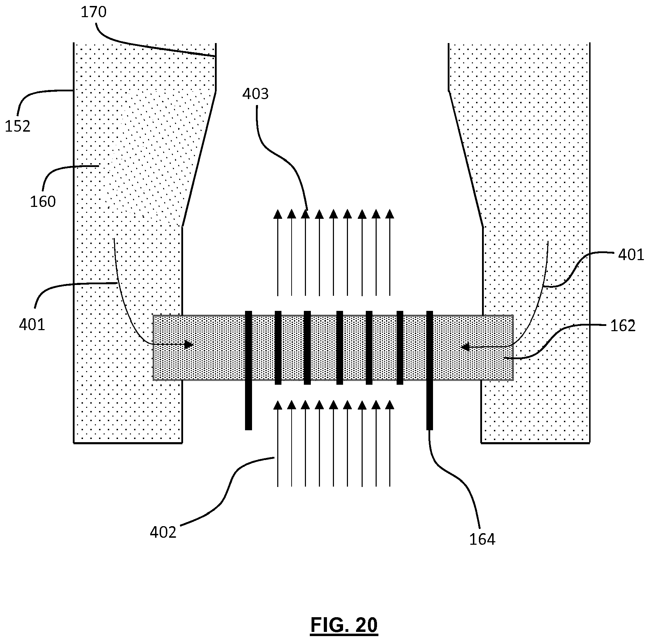

Figure 20 illustrates in more detail the aerosol generation chamber of the reference arrangement as shown in Figure 19 and therefore the region of the consumable 150 around the wick 162 and filament 164. The helical filament 164 is wound around a central portion of the porous wick 162. The porous wick extends across passage 170. E-liquid 160 contained within the tank 152 is conveyed as illustrated schematically by arrows 401, i.e. from the tank and towards the central portion of the porous wick 162.

When the user inhales, air is drawn from through the inlets 176 shown in Figure 19, along inlet flow channel 178 to aerosol generation chamber inlet 172 and into the aerosol generation chamber containing porous wick 162. The porous wick 162 extends substantially transverse to the air flow direction. The air flow passes around the porous wick, at least a portion of the air flow substantially following the surface of the porous wick 162. In examples where the porous wick has a cylindrical cross-sectional profile, the air flow may follow a curved path around an outer periphery of the porous wick 162.

At substantially the same time as the air flow passes around the porous wick 162, the filament 164 is heated so as to vaporise the e-liquid which has been wicked into the porous wick. The air flow passing around the porous wick 162 picks up this vaporised e-liquid, and the vapour-containing air flow is drawn in direction 403 further down passage 170.

The power source of the main body 120 may be in the form of a battery (e.g. a rechargeable battery such as a lithium ion battery). The main body 120 may comprise a connector in the form of e.g. a USB port for recharging this battery. The main body 120 may also comprise a controller that controls the supply of power from the power source to the main body electrical contacts (and thus to the filament 164). That is, the controller may be configured to control a voltage applied across the main body electrical contacts, and thus the voltage applied across the filament 164. In this way, the filament 164 may only be heated under certain conditions (e.g. during a puff and/or only when the system is in an active state). In this respect, the main body 120 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 120 and consumable 150 may comprise a further interface which may, for example, be in the form of an RFID reader, a barcode or QR code reader. This interface may be able to identify a characteristic (e.g. a type) of a consumable 150 engaged with the main body 120. In this respect, the consumable 150 may include any one or more of an RFID chip, a barcode or QR code, or memory within which is an identifier and which can be interrogated via the interface.

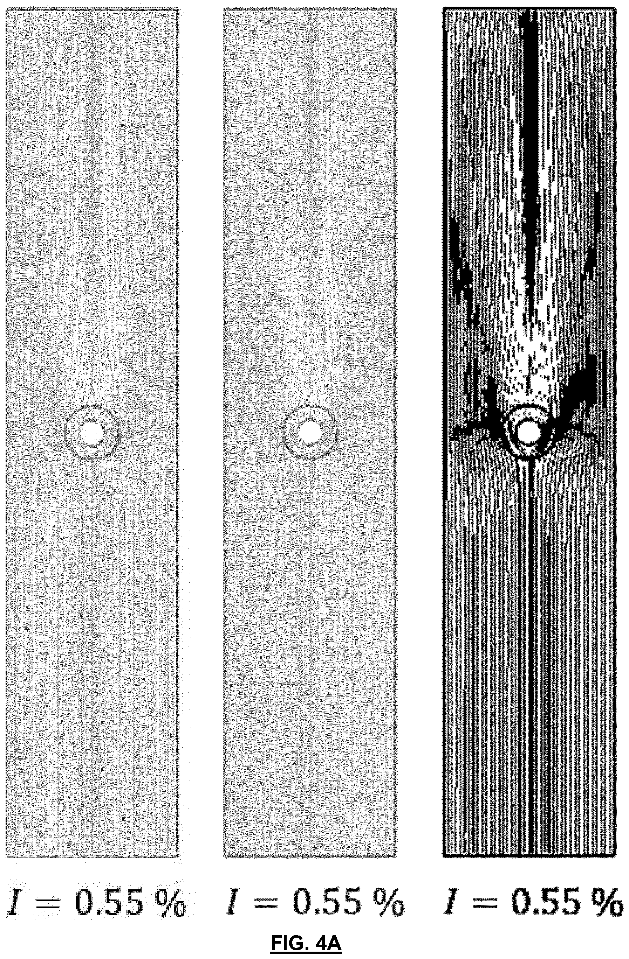

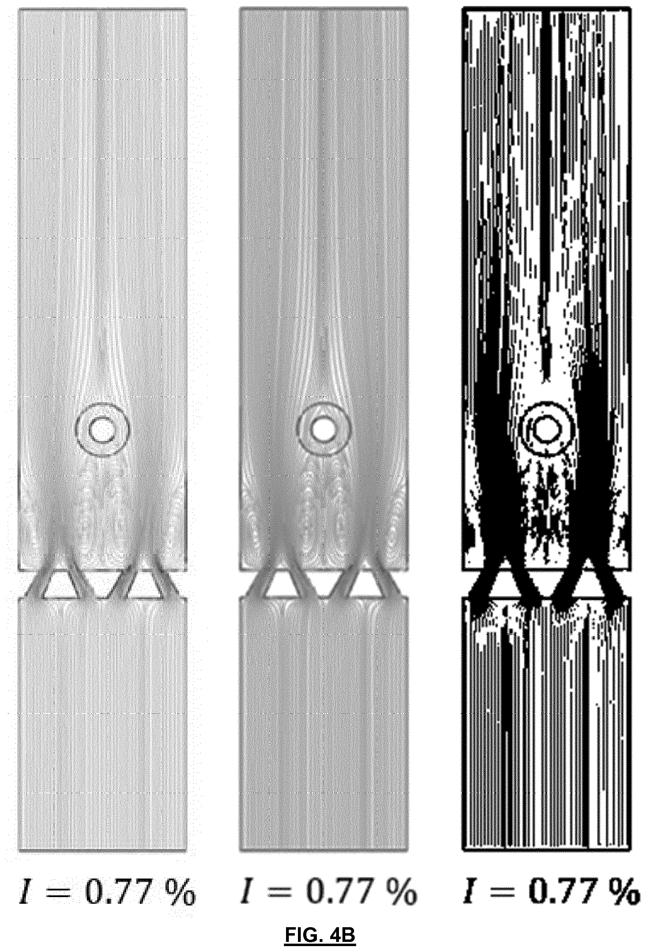

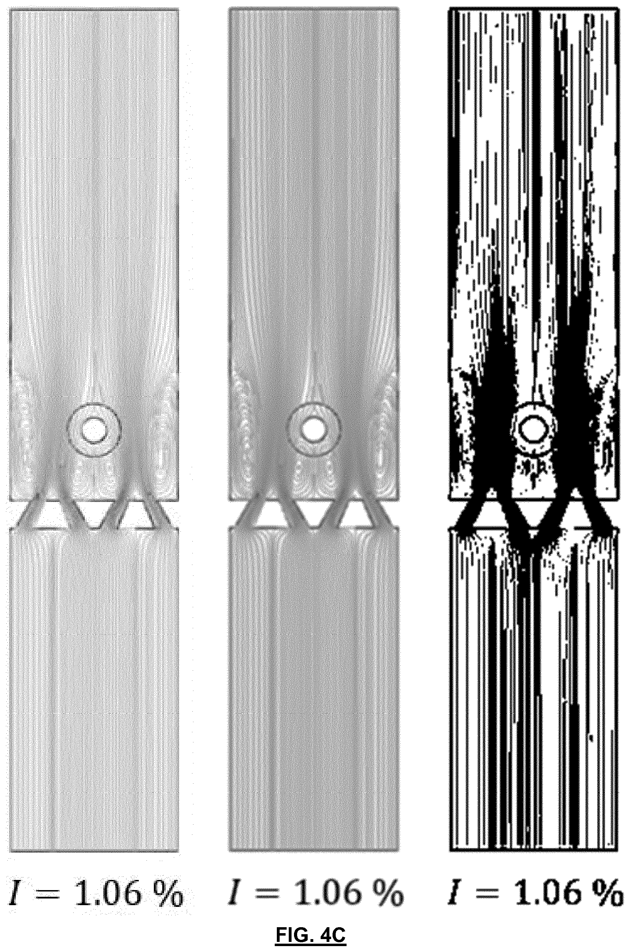

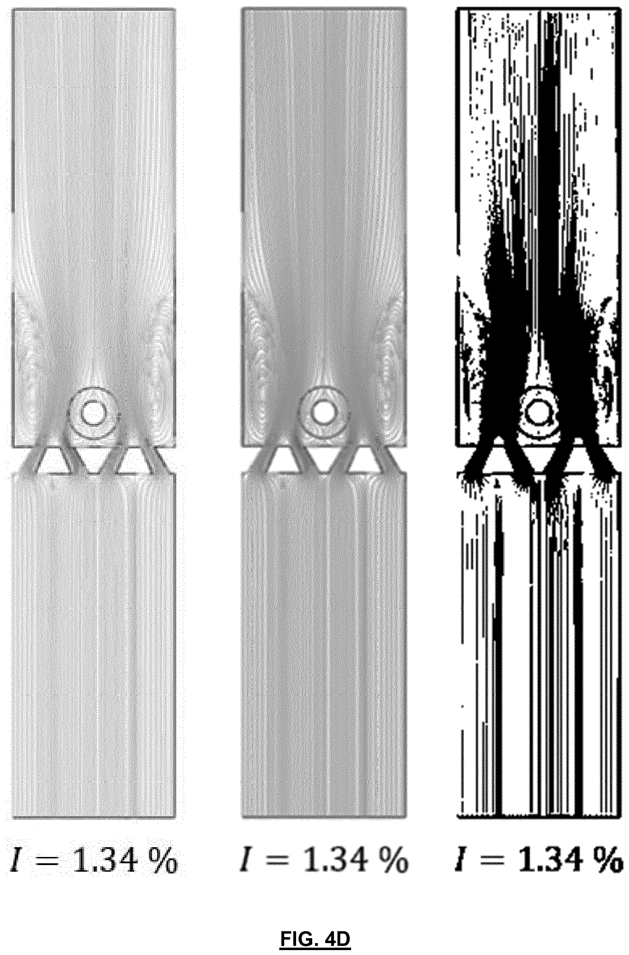

Figures 21A, 21B and 21C respectively illustrate a longitudinal cross sectional view of a consumable 250, an enlarged cross sectional view of a junction of the consumable 250, and an enlarged cross sectional view of the base of the consumable 250 according to the first embodiment of the present disclosure. In Figure 21A, the consumable 250 is shown attached, at a first end of the consumable 250, to the main body 120 of Figure 17 and Figure 18. More specifically, the consumable 250 is configured to engage and disengage with the main body 120 and is interchangeable with the reference arrangement 150 as shown in Figures 3 and 4. Furthermore, the consumable 250 is configured to interact with the main body 120 in the same manner as the reference arrangement 150 and the user may operate the consumable 250 in substantially the same manner as the reference arrangement 150.

The consumable 250 comprises a housing. The housing comprises an aerosol generation chamber 280 for generating an aerosol. The housing has a plurality of air inlets 272 defined at the sidewall of the housing. An outlet 274 is defined at a second end of the consumable 250 that comprises a mouthpiece 254. Passages 270 each extend between the respective air inlet 272 and the outlet 274 to provide flow passages for an air flow 412 as a user puffs on the mouthpiece 254. In the illustrated embodiment, the passages extend from the air inlets 272 towards the first end of the consumable 250 before routing back to towards the outlet 274 at the second end of the consumable 250. The path of the air flow 412 is illustrated in Figure 21A, 21B and 21C. In other embodiments, the passages 270 may extend from the air inlets 272 directly to the outlets 274 without routing towards the first end of consumable 250.

In addition, as best shown in Figure 21C, a pair of auxiliary passages 276 are branched out from their respective passages 270, whereby the air flow entering the housing is split into a first air flow (e.g. continues along the passages 270 and towards the outlet) and a second air flow (e.g. following the auxiliary passages 276 to enter the aerosol generation chamber 280). The auxiliary passages 276 are configured to provide flow passage for a second air flow entering the air inlets 272 to enter the aerosol generation chamber 280 through bleed apertures 284 at a base of the aerosol generation chamber 280. The path of second air flow 416 entering through the bleed apertures 284 is shown in Figures 21A and 21C.

In contrast with the consumable 150 as shown in Figures 19 and 20, the air flow passages 270 of the consumable 250 allow the majority of air flow, or first air flow, 412 to bypass the aerosol generation chamber 280. The relatively smaller second air flow 416 may enter the aerosol generation chamber through the bleed apertures 284 primarily for reducing the amount of partial vacuum (e.g. lowered pressure) within the aerosol generation chamber 280 during a puff, as well as displacing residual aerosol therefrom. Together the first air flow 412 and the second air flow 416 contributes to the total amount of air flow at the outlet. The amount of said second air flow 416 contributes not more than 35% of the air flow at the outlet 274. The amount of said first air flow 412 contributes at least 65% of the air flow at the outlet 274. Such arrangement allows aerosol precursor to be vaporised in presence of a much reduced air flow in comparison to the reference arrangement 150, thus resulting in a much reduced turbulence. The vaporised aerosol precursor may cool and therefore condense to form an aerosol in the aerosol generation chamber 280, which is subsequently drawn into or entrained with the first air flow in passages 270. In addition, a portion of the vaporised aerosol precursor may remain as a vapour before leaving the aerosol generation chamber 280, and subsequently forms an aerosol as it is cooled by the cooler air flow in the passages 270. The flow path of the vapour and/or aerosol 414 is illustrated in Figures 21A and 21B.

As shown in Figures 21A and 21B, the aerosol generation chamber 280 having a chamber outlet 282 opened towards the outlet 274 of the consumable 250. In the illustrated embodiment, the chamber outlet 282 is configured to be in fluid communication with junctions 290 at passages 270 through respective aerosol channels 292. The junction 290 merges the aerosol channels 292 with the passages 270 such that vapour and/or aerosol formed in the aerosol generation chamber 280 may expand or be drawn into the passages 270 through a junction inlet of respective junctions 290. The aerosol channels 292 form a buffering volume to minimise the amount of air flow back-flowing into the aerosol generation chamber 280. In some other embodiments, the chamber outlet 282 directly opens towards the junctions 290 at respective passages 270, and therefore in such embodiments the aerosol channels 292 may be omitted. In the illustrated embodiment, the aerosol generation chamber is configured to have a length of 20mm and a volume of 680mm$^3$. The aerosol generation chamber is configured to allow aerosol to be expulsed through the chamber outlet at a rate greater than 0.1mg/second. In other embodiments the aerosol generation chamber may be configured to have an internal volume ranging between 68mm$^3$ to 680mm$^3$, wherein the length of the aerosol generation chamber may range between 2mm to 20mm.

As shown in Figures 21A and 21B, a part of each of the passages 270 extends alongside a portion of the external sidewalls of the aerosol generation chamber 280. The aerosol generation chamber 280 comprises a heater extending across its width. The heater comprises a porous wick 262 and a heating filament 264 helically wound around a portion of the porous wick 162. A tank 252 is provided in the space between the aerosol generation chamber 280 and the outlet 274 for storing a reservoir of aerosol precursor. Therefore in contrast with the reference arrangement as shown in Figures 19 and 20, the tank 252 in this embodiment does not substantially surround the aerosol generation chamber 280 nor the passages 270. Instead, as shown in Figure 21A, the tank is substantially positioned above the aerosol generation chamber 280 and the porous wick 262 when the consumable 250 is placed in an upright orientation. The end portions of the porous wick 262 extend through the sidewalls of the aerosol generation chamber 280 and into liquid conduits 266 which is in fluid communication with the tank 252. Such arrangement may allow the aerosol precursor stored in the tank 252 to convey towards the porous wick 262 through the liquid conduits 266 by gravity. The liquid conduits 266 are configured to have a hydraulic diameter that allow a controlled amount of aerosol precursor to flow from the tank 252 towards the porous wick 262. More specifically, the size of liquid conduits 266 may be selected based on the rate of aerosol precursor consumption during vaporisation. For example, each of the liquid conduits 266 is sized such that it allows a sufficient amount of aerosol precursor to flow towards and replenish the wick, yet not large enough to cause excessive aerosol precursor to leak into the aerosol generation chamber. The liquid conduits 266 are configured to have a hydraulic diameter ranging from 0.01mm to 10mm or 0.01mm to 5mm. Preferably, the liquid conduits 266 are configured to have a hydraulic diameter in the range of 0.1mm to 1mm.

The heating filament is electrically connected to electrical contacts 256 at the base of the aerosol generation chamber 280. As shown in Figure 21A, when the first end of the consumable 250 is received into a cavity of the main body 120, the electrical contacts 256 are put into electrical communication with corresponding electrical contacts of the main body 120, and thereby allowing the heater to be energised.

The aerosol may discharge from the aerosol generation chamber 280 based on pressure difference between the aerosol generation chamber 280 and the passages 270. Such pressure difference may arise from i) an increased pressure in the aerosol generation chamber 280 during vaporisation of aerosol precursor, ii) the second air flow 416 entering into the aerosol generation chamber 280 through the bleed apertures 284, and/or iii) a reduced pressure in the first air flow along the passages 270 during a puff.

For example, during a user puff the bleed apertures 284 allow a second air flow to enter through the base of the aerosol generation chamber 280 before flowing towards the chamber outlet 282. Such second air flow sweeps the length of the chamber 280 and thereby allows residual aerosol droplets to be evacuated from the aerosol generation chamber 280. Moreover, during or after the user puff the second air flow 416 entering the bleed apertures 284 may help equalising a partial vacuum (e.g. a lowered pressure) in the aerosol generation chamber 280 which could otherwise hold back the generated aerosol in the aerosol generation chamber 280. Thus, such arrangement aids the discharge of generated aerosol through the chamber outlet 282.

In the illustrated embodiment, a pair of bleed apertures 284 are provided across the base of the aerosol generation chamber 280. That is, the second air flow 416 is distributed over an enlarged region at the base and therefore such arrangement may improve the efficiency in evacuating the generated aerosol from the aerosol generation chamber 280. In some other embodiment, the bleed apertures 284 may be arranged in the peripheral region of the base and do not axially align with the heater. Thus, such arrangement may reduce the chance the second air flow 416 directly impinging directly upon the heater as it enter the aerosol generation chamber 280.

The proportion of the first and second air flow splitting respectively between the passages 270 and the auxiliary passages 276 depends upon the relative flow resistance along their respective flow paths. For example, the bleed apertures 284 may have a smaller hydraulic diameter than the passages 270 and therefore results in an elevated flow resistance. Such arrangement limits the second air flow entering the aerosol generation chamber 280 through the bleed apertures 284. The size of the bleed apertures may range between 0.01 to 10mm, or within the range of 0.01mm to 1mm. In the present embodiment, the bleed apertures each measure 1mm in diameter.

Alternatively or in addition, the relative flow resistance between the auxiliary passages 276 and the passages 270 may be varied by changing the relative sizes of the auxiliary passages 276 and/or passages 270. For example, narrower auxiliary passages 276 may be adapted to limit the flowrate of the second air flow therethrough, and therefore accordingly increasing the flowrate of the first air flow bypassing the aerosol generation chamber 280 through the passages 270. In the illustrated embodiment, an air flow amounting to less than 35% of the air flow at the outlet branches out from the passages 270 to enter the aerosol generation chamber through the bleed apertures 284. In some other embodiments, the amount of second air flow entering the aerosol generation chamber 280 through the bleed apertures 284 is less than any one of 30%, 25%, 20%, 15%, 10% and 5% of the air flow at the outlet.

In some embodiments, the auxiliary flow passages 276 and/or the bleed apertures 284 may each be provided with a one way valve, such as a check valve. Said one way valve may provide one way flow passage for the second air flow 416 to enter the aerosol generation chamber 280, as well as keeping aerosol precursor and/or aerosol droplets from leaking, through the bleed apertures 284, into the auxiliary passages 276.

During a puff, the aerosol generated in the aerosol generation chamber 280 may be drawn out from the chamber outlet 282 by a region of lowered pressure downstream created by a Venturi effect. As best illustrated in Figure 21B, junctions 290 each comprises a constriction 294 which formed from a narrowed section of respective passage 270. The flow constrictions 294 are configured to have a smaller cross sectional area than the respective passages 270 upstream, or more specifically immediately upstream, to the junctions 290. In the illustrated embodiment, the passages 270 further comprise widened portions 296 immediately upstream of the flow constrictions 294. Said widened portions 296 are configured to have a larger cross sectional area in comparison to the respective passages 270 upstream.

During a user puff, where the user draws an air flow from air inlets 272 towards the outlet 274, the velocity of the first air flow may reduce in the widened portions 296 due to the enlarged cross sectional area thereat. As the first air flow continues along the passages 270 it converges through the flow constrictions 294 and thereby increases in velocity. According to Bernoulli's principle, as the air flow increases in speed through a constriction, it results in a region of localised pressure drop thereat. As such, as the first air flow passes through junctions 290, it may induce additional suction at the junction inlets for drawing out vapour and/or aerosol from the aerosol generation chamber towards the air flow. Such phenomena may be referred to as the Venturi effect. Advantageously, such arrangement allows vapour and/or aerosol to be withdrawn from the aerosol generation chamber in a more effective manner. The junction inlets at junction 290, as shown in Figure 21B, open in a direction orthogonal to the first air flow. That is, the junction inlets open at a sidewall of the passage. This allows the vapour and/or aerosol from the aerosol generation chamber 280 to entrain into the first air flow at an angle, and thus improving localised mixing of the different streams, as well as encouraging aerosol formation. The aerosol may be fully formed in the first air flow and be drawn out through the outlet at the mouthpiece.

The illustrated embodiment comprises widened portions 296 immediately upstream of the constrictions 294, this enables the first air flow to accelerate from a lowered velocity and thereby creating a larger localised pressure drop across the constrictions 294. However in other embodiments such widened portions may be omitted, e.g. the passages 270 upstream of the constrictions 294 may each be configured to have a substantially uniform cross sectional area.

In some other embodiments, different constrictions 294 may be used. For example, an orifice plate having an aperture with a smaller cross sectional area than the respective passage 270 may be provided at or adjacent to each of the junctions for accelerating the air flow thereacross. Such orifice plates may advantageously reduce the footprint of the constrictions 294.

In the illustrated embodiment, the heater is positioned within the aerosol generation chamber 280, e.g. the heater is spaced from the chamber outlet 282. Such arrangement may reduce or prevent the amount of air flow entering the aerosol generation chamber, and therefore it may minimise the amount of turbulence in the vicinity of the heater. Furthermore, such arrangement may increase the residence time of vapour in the aerosol generation chamber 280, and thereby it may result in the formation of larger aerosol droplets. In some other embodiments, the heater may be positioned adjacent to the chamber outlet and therefore that the path of aerosol 414 from the heater to the chamber outlet 282 is shortened. This may allow aerosol to be drawn into or entrained with the first air flow in a more efficient manner.

With the absence of, or much reduced, air flow in the aerosol generation chamber, the aerosol as generated by the illustrated embodiment has a median droplet size $d_{50}$ of at least $1\mu m$. More preferably, the aerosol as generated by the illustrated embodiment has a median droplet size $d_{50}$ in the range $2\mu m$ to $3\mu m$.

Figure 22 illustrates an enlarged cross sectional view of the base of the consumable 350 according to the second embodiment of the present disclosure. The consumable 350 is similar to consumable 250 of the first embodiment, apart from that the auxiliary passages 376 in consumable 350 each comprises a variable auxiliary flow constrictor 377 for varying the flow resistance thereacross. The variable auxiliary flow constrictors 377 may each comprise one or more of a manually actuated valve, an electronically actuated valve and a deformable auxiliary passage wall. More specifically, the variable auxiliary flow constrictors 377 are configured to vary the cross sectional area along a section of or the entire auxiliary passage 376.

In the illustrated embodiment, the variable auxiliary flow constrictors 377 each comprises a section of the deformable auxiliary passage wall 377, which is actuated by depressing an adjacent section of deformable sidewall at the housing 379. Both sections of deformable auxiliary passage walls 377 and deformable housing sidewalls 379 are formed from an elastomer, such as rubber. In use, a user may press onto the section of deformable housing sidewalls 379, causing them to bias towards and therefore compress their respective deformable auxiliary passage walls 377. This results in a narrowed auxiliary passages 376 with an increased flow resistance, and thereby allowing a larger amount of first air flow 412 to bypass the aerosol generation chamber 380 through the passages 370 during a user puff. Upon releasing the pressure on the deformation housing sidewalls 379, the elastic auxiliary passage walls 377 may return to their original shape and therefore the cross sectional area of the auxiliary passages 376 may expand, thus allowing an increased proportion of air flow to enter the aerosol generation chamber 380 through the bleed apertures 384.

In some other embodiments, the variable auxiliary passage walls 377 may be actuated electronically. For example, the variable auxiliary passage walls 377 may each comprise a piezoelectric element electrically connected to the controller in the main body 120. Such arrangement may allow the section of deformation housing walls to be omitted. To actuate the variable auxiliary passage walls 377, the controller may apply an electrical current across the piezoelectric elements and thereby imparting a mechanical force on their respective deformable passage walls 377. This may result in the narrowing of the auxiliary passages 376 and therefore allowing a larger amount of first air flow 412 to bypass the aerosol generation chamber 380 through the passage 370 during a user puff. In some other embodiment, the variable auxiliary flow constrictors may instead be provided at the bleed apertures for controlling the portion of air flow passing through the bleed apertures. For example, the variable auxiliary flow constrictors may be an electronically actuated valve positioned across the respective bleed apertures. The electronically actuated valves are electrically connected to the controller of the main body 120. In use, the controller controls the degree of opening at the electronically actuated valves in order to vary the hydraulic diameter of the bleed apertures.

There now follows a disclosure of certain experimental work undertaken to determine the effects of certain conditions in the smoking substitute apparatus on the particle size of the generated aerosol.

These experimental results are relevant to the embodiments disclosed above in that they show the effect of altering the flow conditions at the wick on the particle size of the generated aerosol.

## 1. Introduction

Aerosol droplet size is a considered to be an important characteristic for smoking substitution devices. Droplets in the range of 2-5 $\mu$m are preferred in order to achieve improved nicotine delivery efficiency and to minimise the hazard of second-hand smoking. However, at the time of writing (September 2019), commercial EVP devices typically deliver aerosols with droplet size averaged around 0.5 $\mu$m, and to the knowledge of the inventors not a single commercially available device can deliver an aerosol with an average particle size exceeding 1 $\mu$m.

The present inventors speculate, without themselves wishing to be bound by theory, that there has to date been a lack of understanding in the mechanisms of e-liquid evaporation, nucleation and droplet growth in the context of aerosol generation in smoking substitute devices. The present inventors have therefore studied these issues in order to provide insight into mechanisms for the generation of aerosols with larger particles. The present inventors have carried out experimental and modelling work alongside theoretical investigations, leading to significant achievements as now reported.

This disclosure considers the roles of air velocity, air turbulence and vapour cooling rate in affecting aerosol particle size.

## 2. Experiments

In this work, a Malvern PANalytical Spraytec laser diffraction system was employed for the particle size measurement. In order to limit the number of variables, the same coil and wick (1.5 ohms Ni-Cr coil, 1.8 mm Y07 cotton wick), the same e-liquid (1.6% freebase nicotine, 65:35 propylene glycol (PG)/vegetable glycerine (VG) ratio, no added flavour) and the same input power (10W) were used in all experiments. Y07 represents the grade of cotton wick, meaning that the cotton has a linear density of 0.7 grams per meter.

Particle sizes were measured in accordance with ISO 13320:2009(E), which is an international standard on laser diffraction methods for particle size analysis. This is particularly well suited to aerosols, because there is an assumption in this standard that the particles are spherical (which is a good assumption for liquid-based aerosols). The standard is stated to be suitable for particle sizes in the range 0.1 micron to 3 mm.

The results presented here concentrate on the volume-based median particle size Dv50. This is to be taken to be the same as the parameter $d_{50}$ used above.

## 2.1. Rectangular tube testing

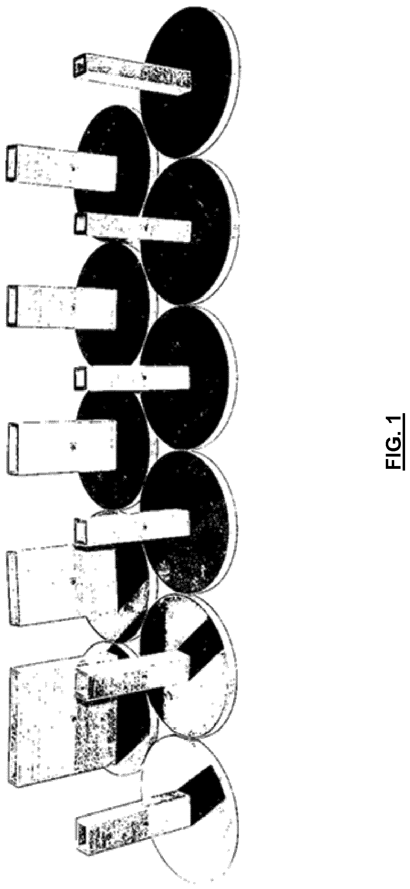

The work reported here based on the inventors' insight that aerosol particle size might be related to: 1) air velocity; 2) flow rate; and 3) Reynolds number. In a given EVP device, these three parameters are interlinked to each other, making it difficult to draw conclusions on the roles of each individual factor. In order to decouple these factors, experiments were carried out using a set of rectangular tubes having different dimensions. These were manufactured by 3D printing. The rectangular tubes were 3D printed in an MJP 2500 3D printer. Figure 1 illustrates the set of rectangular tubes. Each tube has the same depth and length but different width. Each tube has an integral end plate in order to provide a seal against air flow outside the tube. Each tube also has holes formed in opposing side walls in order to accommodate a wick.

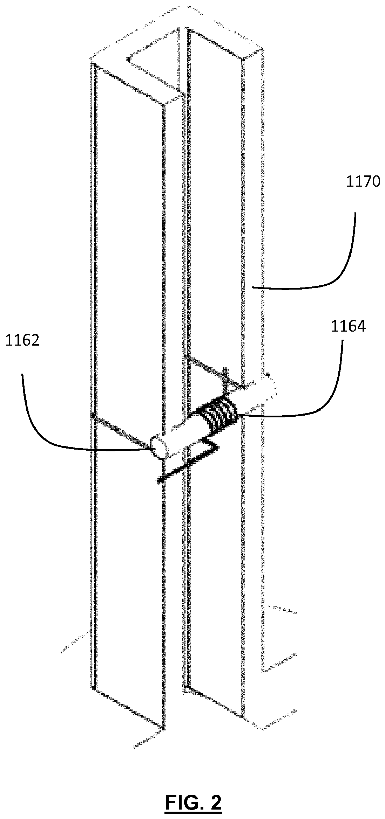

Figure 2 shows a schematic perspective longitudinal cross sectional view of an example rectangular tube 1170 with a wick 1162 and heater coil 1164 installed. The location of the wick is about half way along the length of the tube. This is intended to allow the flow of air along the tube to settle before reaching the wick.

Figure 3 shows a schematic transverse cross sectional view an example rectangular tube 1170 with a wick 1162 and heater coil 1164 installed. In this example, the internal width of the tube is 12 mm

The rectangular tubes were manufactured to have same internal depth of 6 mm in order to accommodate the standardized coil and wick, however the tube internal width varied from 4.5 mm to 50 mm. In this disclosure, the "tube size" is referred to as the internal width of rectangular tubes.

The rectangular tubes with different dimensions were used to generate aerosols that were tested for particle size in a Malvern PANalytical Spraytec laser diffraction system. An external digital power supply was dialled to 2.6A constant current to supply 10W power to the heater coil in all experiments. Between two runs, the wick was saturated manually by applying one drop of e-liquid on each side of the wick.

Three groups of experiments were carried out in this study:

1. 1.3 lpm (litres per minute, L min-1 or LPM) constant flow rate on different size tubes
2. 2.0 lpm constant flow rate on different size tubes
3. 1 m/s constant air velocity on 3 tubes: i) 5mm tube at 1.4 lpm flow rate; ii) 8mm tube at 2.8 lpm flow rate; and iii) 20mm tube at 8.6 lpm flow rate.

Table 1 shows a list of experiments in this study. The values in "calculated air velocity" column were obtained by simply dividing the flow rate by the intersection area at the centre plane of wick. Reynolds numbers (Re) were calculated through the following equation:

$$Re = \frac{\rho v L}{\mu}$$

where: $\rho$ is the density of air (1.225 kg/m$^3$); $v$ is the calculated velocity in table 1; $\mu$ is the viscosity of air (1.48 $\times$ 10$^{-5}$ m$^2$/s); $L$ is the characteristic length calculated by:

$$L = \frac{4P}{A}$$

where: P is the perimeter of the flow path's intersection, and A is the area of the flow path's intersection.

*Table 1. List of experiments in the rectangular tube study*

|  | Tube size [mm] | Flow rate [lpm] | Reynolds number | Calculated air velocity [m/s] |
|---|---|---|---|---|
| 1.3 lpm constant flow rate | 4.5 | 1.3 | 153 | 1.17 |
|  | 6 | 1.3 | 142 | 0.71 |
|  | 7 | 1.3 | 136 | 0.56 |
|  | 8 | 1.3 | 130 | 0.47 |
|  | 10 | 1.3 | 120 | 0.35 |
|  | 12 | 1.3 | 111 | 0.28 |
|  | 20 | 1.3 | 86 | 0.15 |
|  | 50 | 1.3 | 47 | 0.06 |

(continued)

|  | Tube size [mm] | Flow rate [lpm] | Reynolds number | Calculated air velocity [m/s] |
|---|---|---|---|---|
| 2.0 lpm constant flow rate | 4.5 | 2.0 | 236 | 1.81 |
| | 5 | 2.0 | 230 | 1.48 |
| | 6 | 2.0 | 219 | 1.09 |
| | 8 | 2.0 | 200 | 0.72 |
| | 12 | 2.0 | 171 | 0.42 |
| | 20 | 2.0 | 132 | 0.23 |
| | 50 | 2.0 | 72 | 0.09 |
| 1.0 m/s constant air velocity | 5.0 | 1.4 | 155 | 1.00 |
| | 8 | 2.8 | 279 | 1.00 |
| | 20 | 8.6 | 566 | 1.00 |

Five repetition runs were carried out for each tube size and flow rate combination. Between adjacent runs there were at least 5 minutes wait time for the Spraytec system to be purged. In each run, real time particle size distributions were measured in the Spraytec laser diffraction system at a sampling rate of 2500 per second, the volume distribution median (Dv50) was averaged over a puff duration of 4 seconds. Measurement results were averaged and the standard deviations were calculated to indicate errors as shown in section 4 below.

2.2. Turbulence tube testing

The Reynolds numbers in Table 1 are all well below 1000, therefore, it is considered fair to assume all the experiments in section 2.1 would be under conditions of laminar flow. Further experiments were carried out and reported in this section to investigate the role of turbulence.

Turbulence intensity was introduced as a quantitative parameter to assess the level of turbulence. The definition and simulation of turbulence intensity is discussed below (see section 3.2).

Different device designs were considered in order to introduce turbulence. In the experiments reported here, jetting panels were added in the existing 12mm rectangular tubes upstream of the wick. This approach enables direct comparison between different devices as they all have highly similar geometry, with turbulence intensity being the only variable.

Figures 4A-4D show air flow streamlines in the four devices used in this turbulence study. Figure 4A is a standard 12mm rectangular tube with wick and coil installed as explained in the previous section, with no jetting panel. Figure 4B has a jetting panel located 10mm below (upstream from) the wick. Figure 4C has the same jetting panel 5mm below the wick. Figure 4D has the same jetting panel 2.5mm below the wick. As can be seen from Figures 4B-4D, the jetting panel has an arrangement of apertures shaped and directed in order to promote jetting from the downstream face of the panel and therefore to promote turbulent flow. Accordingly, the jetting panel can introduce turbulence downstream, and the panel causes higher level of turbulence near the wick when it is positioned closer to the wick. As shown in Figures 4A-4D, the four geometries gave turbulence intensities of 0.55%, 0.77%, 1.06% and 1.34%, respectively, with Figure 4A being the least turbulent, and Figure 4D being the most turbulent.

For each of Figures 4A-4D, there are shown three modelling images. The image on the left shows the original image (colour in the original), the central image shows a greyscale version of the image and the right hand image shows a black and white version of the image. As will be appreciated, each version of the image highlights slightly different features of the flow. Together, they give a reasonable picture of the flow conditions at the wick.

These four devices were operated to generate aerosols following the procedure explained above (section 2.1) using a flow rate of 1.3 lpm and the generated aerosols were tested for particle size in the Spraytec laser diffraction system.

2.3. High temperature testing

This experiment aimed to investigate the influence of inflow air temperature on aerosol particle size, in order to investigate the effect of vapour cooling rate on aerosol generation.

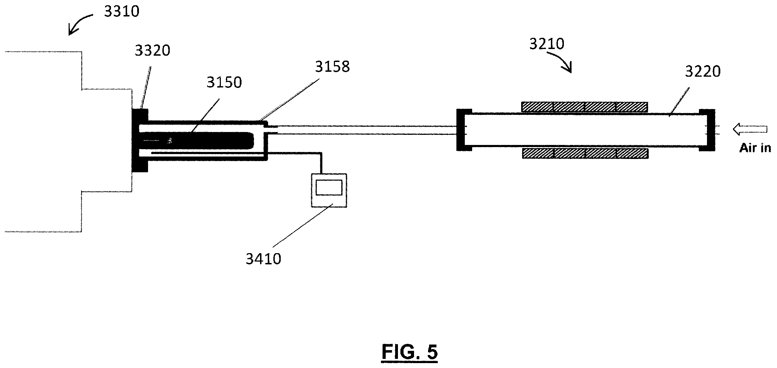

The experimental set up is shown in Figure 5. The testing used a Carbolite Gero EHA 12300B tube furnace 3210 with a quartz tube 3220 to heat up the air. Hot air in the tube furnace was then led into a transparent housing 3158 that contains the EVP device 3150 to be tested. A thermocouple meter 3410 was used to assess the temperature of the air pulled into the EVP device. Once the EVP device was activated, the aerosol was pulled into the Spraytec laser diffraction system 3310 via a silicone connector 3320 for particle size measurement.

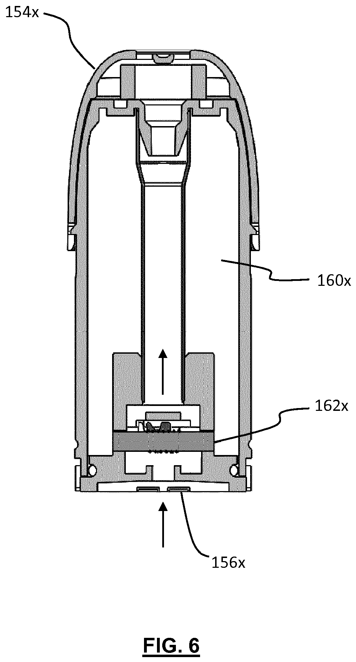

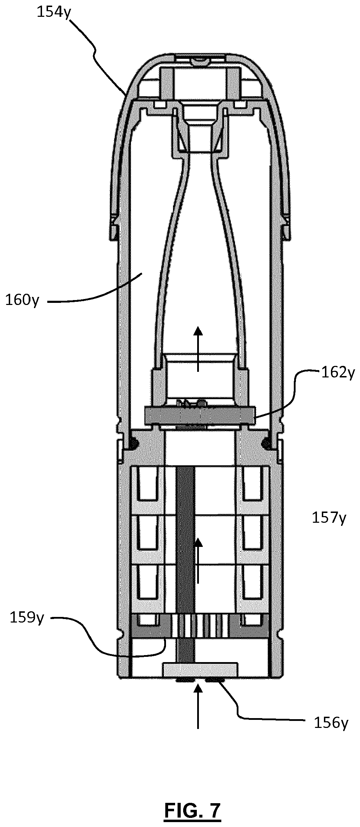

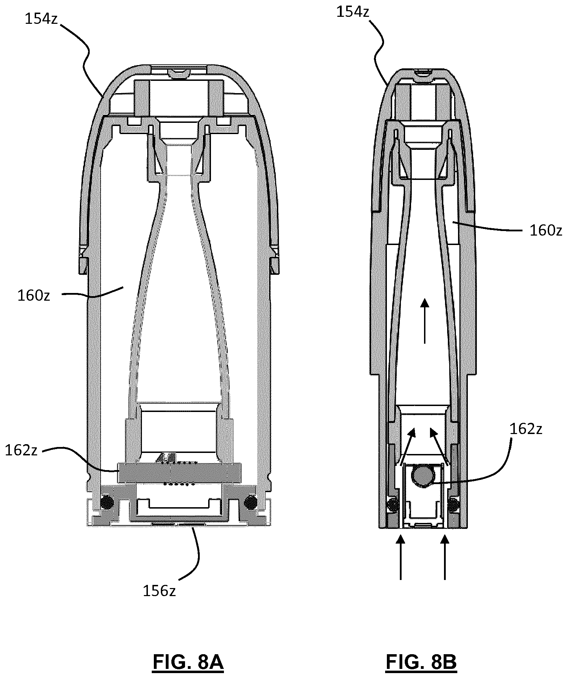

Three smoking substitute apparatuses (referred to as "pods") were tested in the study: pod 1 is the commercially available "myblu optimised" pod (Figure 6); pod 2 is a pod featuring an extended inflow path upstream of the wick (Figure 7); and pod 3 is pod with the wick located in a stagnant vaporisation chamber and the inlet air bypassing the vaporisation chamber but entraining the vapour from an outlet of the vaporisation chamber (Figures 8A and 8B).

Pod 1, shown in longitudinal cross sectional view (in the width plane) in Figure 6, has a main housing that defines a tank 160x holding an e-liquid aerosol precursor. Mouthpiece 154x is formed at the upper part of the pod. Electrical contacts 156x are formed at the lower end of the pod. Wick 162x is held in a vaporisation chamber. The air flow direction is shown using arrows.

Pod 2, shown in longitudinal cross sectional view (in the width plane) in Figure 7, has a main housing that defines a tank 160y holding an e-liquid aerosol precursor. Mouthpiece 154y is formed at the upper part of the pod. Electrical contacts 156y are formed at the lower end of the pod. Wick 162y is held in a vaporisation chamber. The air flow direction is shown using arrows. Pod 2 has an extended inflow path (plenum chamber 157y) with a flow conditioning element 159y, configured to promote reduced turbulence at the wick 162y.

Figure 8A shows a schematic longitudinal cross sectional view of pod 3. Figure 8B shows a schematic longitudinal cross sectional view of the same pod 3 in a direction orthogonal to the view taken in Figure 8A. Pod 3 has a main housing that defines a tank 160z holding an e-liquid aerosol precursor. Mouthpiece 154z is formed at the upper part of the pod. Electrical contacts 156z are formed at the lower end of the pod. Wick 162z is held in a vaporisation chamber. The air flow direction is shown using arrows. Pod 3 uses a stagnant vaporiser chamber, with the air inlets bypassing the wick and picking up the vapour/aerosol downstream of the wick.

All three pods were filled with the same e-liquid (1.6% freebase nicotine, 65:35 PG/VG ratio, no added flavour). Three experiments were carried out for each pod: 1) standard measurement in ambient temperature; 2) only the inlet air was heated to 50 °C; and 3) both the inlet air and the pods were heated to 50 °C. Five repetition runs were carried out for each experiment and the Dv50 results were taken and averaged.

3. Modelling work

In this study, modelling work was performed using COMSOL Multiphysics 5.4, engaged physics include: 1) laminar single-phase flow; 2) turbulent single-phase flow; 3) laminar two-phase flow; 4) heat transfer in fluids; and (5) particle tracing. Data analysis and data visualisation were mostly completed in MATLAB R2019a.

3.1. Velocity modelling

Air velocity in the vicinity of the wick is believed to play an important role in affecting particle size. In section 2.1, the air velocity was calculated by dividing the flow rate by the intersection area, which is referred to as "calculated velocity" in this work. This involves a very crude simplification that assumes velocity distribution to be homogeneous across the intersection area.

In order to increase reliability of the work, computational fluid dynamics (CFD) modelling was performed to obtain more accurate velocity values:

1) The average velocity in the vicinity of the wick (defined as a volume from the wick surface to 1mm away from the wick surface)

2) The maximum velocity in the vicinity of the wick (defined as a volume from the wick surface to 1mm away from the wick surface)

*Table 2. Average and maximum velocity in the vicinity of wick surface obtained from CFD modelling*

| | Tube size [mm] | Flow rate [lpm] | Calculated velocity* [m/s] | Average velocity** [m/s] | Maximum Velocity** [m/s] |
|---|---|---|---|---|---|
| 1.3 lpm constant flow rate | 4.5 | 1.3 | 1.17 | 0.99 | 1.80 |
| | 6 | 1.3 | 0.71 | 0.66 | 1.22 |
| | 7 | 1.3 | 0.56 | 0.54 | 1.01 |
| | 8 | 1.3 | 0.47 | 0.46 | 0.86 |
| | 10 | 1.3 | 0.35 | 0.35 | 0.66 |
| | 12 | 1.3 | 0.28 | 0.27 | 0.54 |
| | 20 | 1.3 | 0.15 | 0.15 | 0.32 |
| | 50 | 1.3 | 0.06 | 0.05 | 0.12 |
| 2.0 lpm constant flow rate | 4.5 | 2.0 | 1.81 | 1.52 | 2.73 |
| | 5 | 2.0 | 1.48 | 1.31 | 2.39 |
| | 6 | 2.0 | 1.09 | 1.02 | 1.87 |
| | 8 | 2.0 | 0.72 | 0.71 | 1.31 |
| | 12 | 2.0 | 0.42 | 0.44 | 0.83 |
| | 20 | 2.0 | 0.23 | 0.24 | 0.49 |
| | 50 | 2.0 | 0.09 | 0.08 | 0.19 |
| * Calculated by dividing flow rate with intersection area<br>** Obtained from CFD modelling | | | | | |

The CFD model uses a laminar single-phase flow setup. For each experiment, the outlet was configured to a corresponding flowrate, the inlet was configured to be pressure-controlled, the wall conditions were set as "no slip". A 1 mm wide ring-shaped domain (wick vicinity) was created around the wick surface, and domain probes were implemented to assess the average and maximum magnitudes of velocity in this ring-shaped wick vicinity domain.

The CFD model outputs the average velocity and maximum velocity in the vicinity of the wick for each set of experiments carried out in section 2.1. The outcomes are reported in Table 2.

3.2. Turbulence modelling

Turbulence intensity (I) is a quantitative value that represents the level of turbulence in a fluid flow system. It is defined as the ratio between the root-mean-square of velocity fluctuations, *u'*, and the Reynolds-averaged mean flow velocity, U:

$$I = \frac{u'}{U} = \frac{\sqrt{\frac{1}{3}\left(u'^2_x + u'^2_y + u'^2_z\right)}}{\sqrt{\overline{u_x}^2 + \overline{u_y}^2 + \overline{u_z}^2}} = \frac{\sqrt{\frac{1}{3}\left[\left(u_x - \overline{u_x}\right)^2 + \left(u_y - \overline{u_y}\right)^2 + +\left(u_z - \overline{u_z}\right)^2\right]}}{\sqrt{\overline{u_x}^2 + \overline{u_y}^2 + \overline{u_z}^2}}$$

where $u_x$, $u_y$ and $u_z$ are the x-, y- and z-components of the velocity vector, $\overline{u_x}$, $\overline{u_y}$, and $\overline{u_z}$ represent the average velocities along three directions.

Higher turbulence intensity values represent higher levels of turbulence. As a rule of thumb, turbulence intensity below 1% represents a low-turbulence case, turbulence intensity between 1% and 5% represents a medium-turbulence case, and turbulence intensity above 5% represents a high-turbulence case.

In this study, turbulence intensity was obtained from CFD simulation using turbulent single-phase setup in COMSOL Multiphysics. For each of the four experiments explained in section 2.2, the outlet was set to 1.3 lpm, the inlet

was set to be pressure-controlled, and all wall conditions were set to be "no slip".

Turbulence intensity was assessed within the volume up to 1 mm away from the wick surface (defined as the wick vicinity domain). For the four experiments explained in section 2.2, the turbulence intensities are 0.55%, 0.77%, 1.06% and 1.34%, respectively, as also shown in Figures 4A-4D.

3.3. Cooling rate modelling

The cooling rate modelling involves three coupling models in COMSOL Multiphysics: 1) laminar two-phase flow; 2) heat transfer in fluids, and 3) particle tracing. The model is setup in three steps:

1) Set up two phase flow model

Laminar mixture flow physics was selected in this study. The outlet was configured in the same way as in section 3.1. However, this model includes two fluid phases released from two separate inlets: the first one is the vapour released from wick surface, at an initial velocity of 2.84 cm/s (calculated based on 5 mg total particulate mass over 3 seconds puff duration) with initial velocity direction normal to the wick surface; the second inlet is air influx from the base of tube, the rate of which is pressure-controlled.

2) Set up two-way coupling with heat transfer physics

The inflow and outflow settings in heat transfer physics was configured in the same way as in the two-phase flow model. The air inflow was set to 25 °C, and the vapour inflow was set to 209 °C (boiling temperature of the e-liquid formulation). In the end, the heat transfer physics is configured to be two-way coupled with the laminar mixture flow physics. The above model reaches steady state after approximately 0.2 second with a step size of 0.001 second.

3) Set up particle tracing

A wave of 2000 particles were release from wick surface at t = 0.3 second after the two-phase flow and heat transfer model has stabilised. The particle tracing physics has one-way coupling with the previous model, which means the fluid flow exerts dragging force on the particles, whereas the particles do not exert counterforce on the fluid flow. Therefore, the particles function as moving probes to output vapour temperature at each timestep.

The model outputs average vapour temperature at each time steps. A MATLAB script was then created to find the time step when the vapour cools to a target temperature (50°C or 75°C), based on which the vapour cooling rates were obtained (Table 3).

*Table 3. Average vapour cooling rate obtained from Multiphysics modelling*

| | Tube size [mm] | Flow rate [lpm] | Cooling rate to 50°C [°C/ms] | Cooling rate to 75°C [°C/ms] |
|---|---|---|---|---|
| 1.3 lpm constant flow rate | 4.5 | 1.3 | 11.4 | 44.7 |
| | 6 | 1.3 | 5.48 | 14.9 |
| | 7 | 1.3 | 3.46 | 7.88 |
| | 8 | 1.3 | 2.24 | 5.15 |
| | 10 | 1.3 | 1.31 | 2.85 |
| | 12 | 1.3 | 0.841 | 1.81 |
| | 20 | 1.3 | 0* | 0.536 |
| | 50 | 1.3 | 0 | 0 |

(continued)

| | Tube size [mm] | Flow rate [lpm] | Cooling rate to 50°C [°C/ms] | Cooling rate to 75°C [°C/ms] |
|---|---|---|---|---|
| 2.0 lpm constant flow rate | 4.5 | 2.0 | 19.9 | 670 |
| | 5 | 2.0 | 13.3 | 67 |
| | 6 | 2.0 | 8.83 | 26.8 |
| | 8 | 2.0 | 3.61 | 8.93 |
| | 12 | 2.0 | 1.45 | 3.19 |
| | 20 | 2.0 | 0.395 | 0.761 |
| | 50 | 2.0 | 0 | 0 |
| * Zero cooling rate when the average vapour temperature is still above target temperature after 0.5 second | | | | |

4. Results and discussions

Particle size measurement results for the rectangular tube testing are shown in Table 4. For every tube size and flow rate combination, five repetition runs were carried out in the Spraytec laser diffraction system. The Dv50 values from five repetition runs were averaged, and the standard deviations were calculated to indicate errors, as shown in Table 4.

In this section, the roles of different factors affecting aerosol particle size will be discussed based on experimental and modelling results.

*Table 4. Particle size measurement results for the rectangular tube testing*

| | Tube size [mm] | Flow rate [lpm] | Dv50 average [μm] | Dv50 standard deviation [μm] |
|---|---|---|---|---|
| 1.3 lpm constant flow rate | 4.5 | 1.3 | 0.971 | 0.125 |
| | 6 | 1.3 | 1.697 | 0.341 |
| | 7 | 1.3 | 2.570 | 0.237 |
| | 8 | 1.3 | 2.705 | 0.207 |
| | 10 | 1.3 | 2.783 | 0.184 |
| | 12 | 1.3 | 3.051 | 0.325 |
| | 20 | 1.3 | 3.116 | 0.354 |
| | 50 | 1.3 | 3.161 | 0.157 |
| 2.0 lpm constant flow rate | 4.5 | 2.0 | 0.568 | 0.039 |
| | 5 | 2.0 | 0.967 | 0.315 |
| | 6 | 2.0 | 1.541 | 0.272 |
| | 8 | 2.0 | 1.646 | 0.363 |
| | 12 | 2.0 | 3.062 | 0.153 |
| | 20 | 2.0 | 3.566 | 0.260 |
| | 50 | 2.0 | 3.082 | 0.440 |
| 1.0 m/s constant air velocity | 5.0 | 1.4 | 1.302 | 0.187 |
| | 8 | 2.8 | 1.303 | 0.468 |
| | 20 | 8.6 | 1.463 | 0.413 |

4.1. Decouple the factors affecting particle size

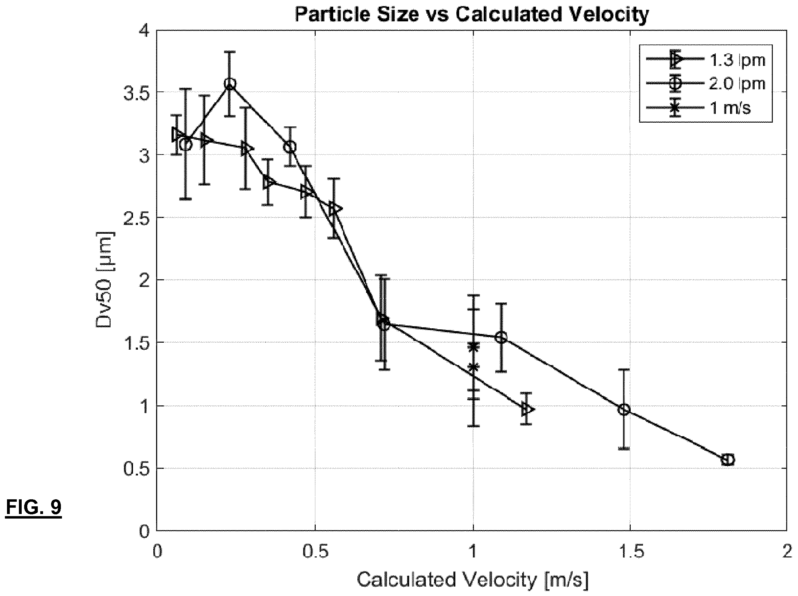

The particle size (Dv50) experimental results are plotted against calculated air velocity in Figure 9. The graph shows a strong correlation between particle size and air velocity.

Different size tubes were tested at two flow rates: 1.3 lpm and 2.0 lpm. Both groups of data show the same trend that slower air velocity leads to larger particle size. The conclusion was made more convincing by the fact that these two groups of data overlap well in Figure 9: for example, the 6mm tube delivered an average Dv50 of 1.697 $\mu$m when tested at 1.3 lpm flow rate, and the 8mm tube delivered a highly similar average Dv50 of 1.646 $\mu$m when tested at 2.0 lpm flow rate, as they have similar air velocity of 0.71 and 0.72 m/s, respectively.

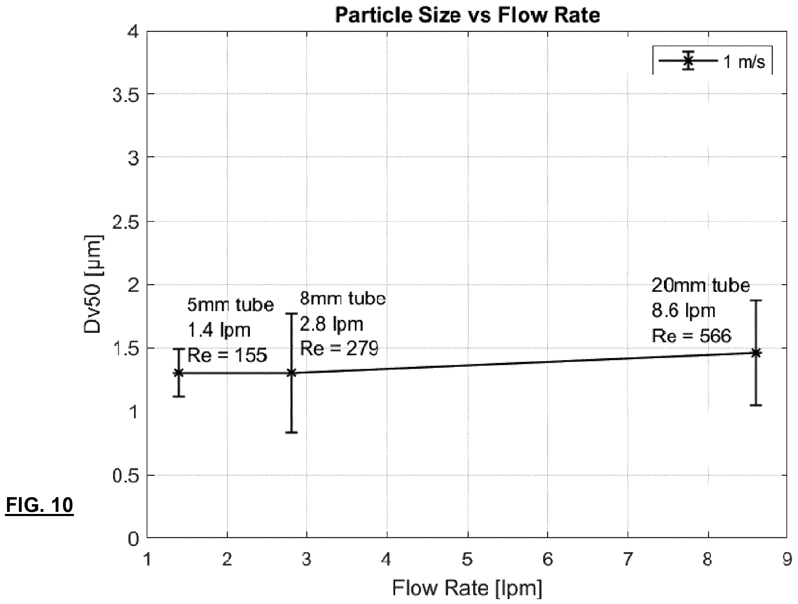

In addition, Figure 10 shows the results of three experiments with highly different setup arrangements: 1) 5mm tube measured at 1.4 lpm flow rate with Reynolds number of 155; 2) 8mm tube measured at 2.8 lpm flow rate with Reynolds number of 279; and 3) 20mm tube measured at 8.6 lpm flow rate with Reynolds number of 566. It is relevant that these setup arrangements have one similarity: the air velocities are all calculated to be 1 m/s. Figure 10 shows that, although these three sets of experiments have different tube sizes, flow rates and Reynolds numbers, they all delivered similar particle sizes, as the air velocity was kept constant. These three data points were also plotted out in Figure 9 (1 m/s data with star marks) and they tie in nicely into particle size-air velocity trendline.

The above results lead to a strong conclusion that air velocity is an important factor affecting the particle size of EVP devices. Relatively large particles are generated when the air travels with slower velocity around the wick. It can also be concluded that flow rate, tube size and Reynolds number are not necessarily independently relevant to particle size, providing the air velocity is controlled in the vicinity of the wick.

4.2. Further consideration of velocity

In Figure 9 the "calculated velocity" was obtained by dividing the flow rate by the intersection area, which is a crude simplification that assumes a uniform velocity field. In order to increase reliability of the work, CFD modelling has been performed to assess the average and maximum velocities in the vicinity of the wick. In this study, the "vicinity" was defined as a volume from the wick surface up to 1 mm away from the wick surface.

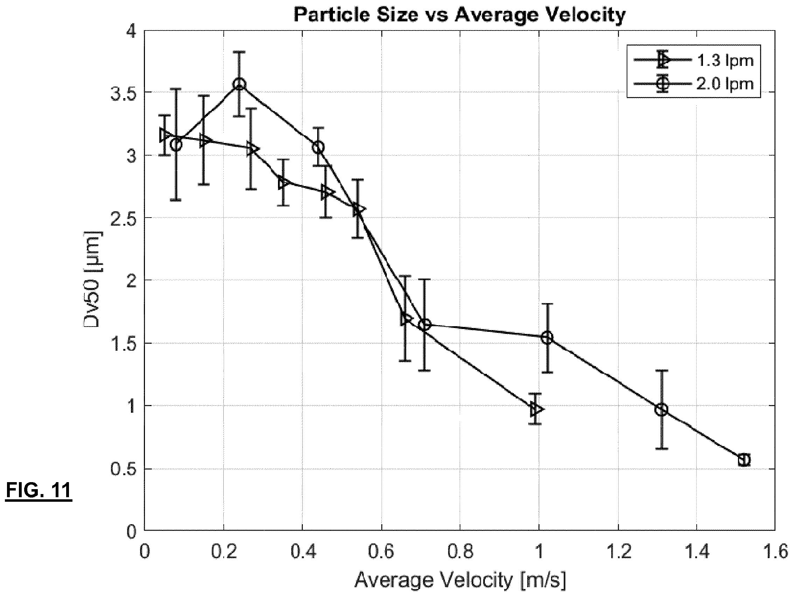

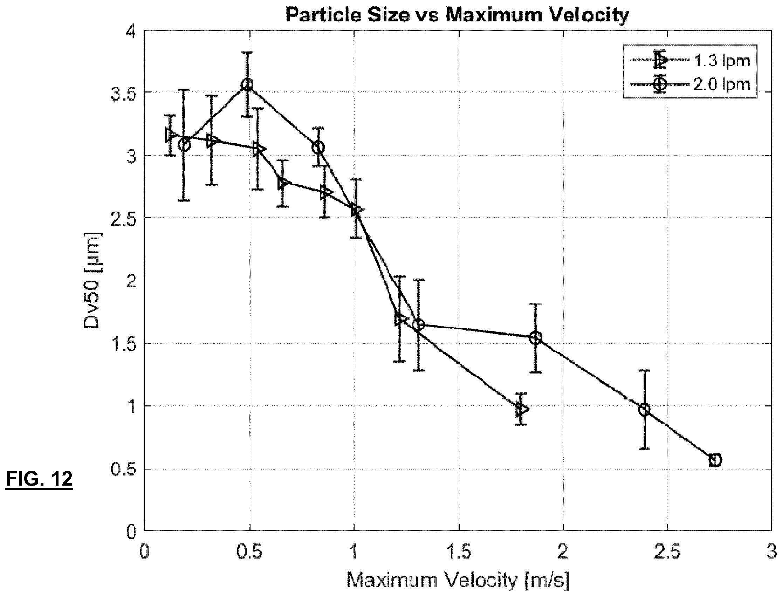

The particle size measurement data were plotted against the average velocity (Figure 11) and maximum velocity (Figure 12) in the vicinity of the wick, as obtained from CFD modelling.

The data in these two graphs indicates that in order to obtain an aerosol with Dv50 larger than 1 $\mu$m, the average velocity should be less than or equal to 1.2 m/s in the vicinity of the wick and the maximum velocity should be less than or equal to 2.0 m/s in the vicinity of the wick.

Furthermore, in order to obtain an aerosol with Dv50 of 2 $\mu$m or larger, the average velocity should be less than or equal to 0.6 m/s in the vicinity of the wick and the maximum velocity should be less than or equal to 1.2 m/s in the vicinity of the wick.

It is considered that typical commercial EVP devices deliver aerosols with Dv50 around 0.5 $\mu$m, and there is no commercially available device that can deliver aerosol with Dv50 exceeding 1 $\mu$m. It is considered that typical commercial EVP devices have average velocity of 1.5-2.0 m/s in the vicinity of the wick.

4.3. The role of turbulence

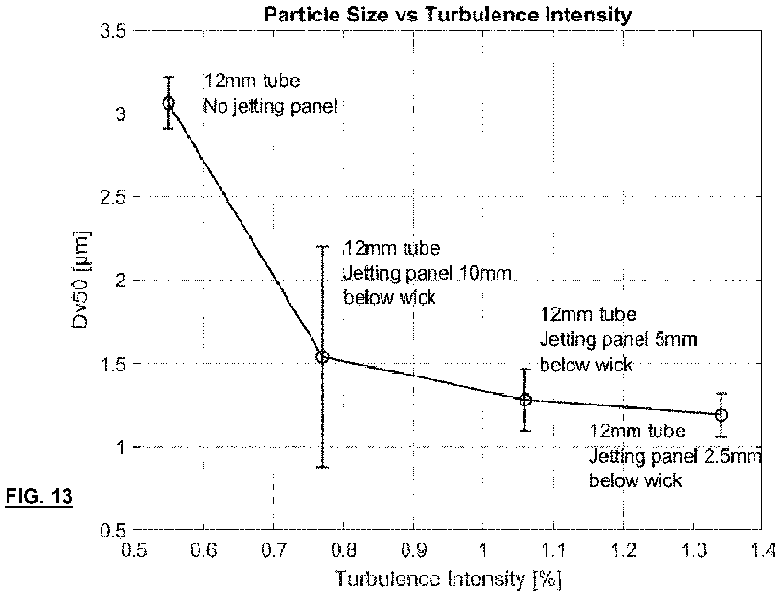

The role of turbulence has been investigated in terms of turbulence intensity, which is a quantitative characteristic that indicates the level of turbulence. In this work, four tubes of different turbulence intensities were used to general aerosols which were measured in the Spraytec laser diffraction system. The particle size (Dv50) experimental results are plotted against turbulence intensity in Figure 13.

The graph suggests a correlation between particle size and turbulence intensity, that lower turbulence intensity is beneficial for obtaining larger particle size. It is noted that when turbulence intensity is above 1% (medium-turbulence case), there are relatively large measurement fluctuations. In Figure 13, the tube with a jetting panel 10mm below the wick has the largest error bar, because air jets become unpredictable near the wick after traveling through a long distance.

The results clearly indicate that laminar air flow is favourable for the generation of aerosols with larger particles, and that the generation of large particle sizes is jeopardised by introducing turbulence. In Figure 13, the 12mm standard rectangular tube (without jetting panel) delivers above 3 $\mu$m particle size (Dv50). The particle size values reduced by at least a half when jetting panels were added to introduce turbulence.

4.4. Vapour cooling rate

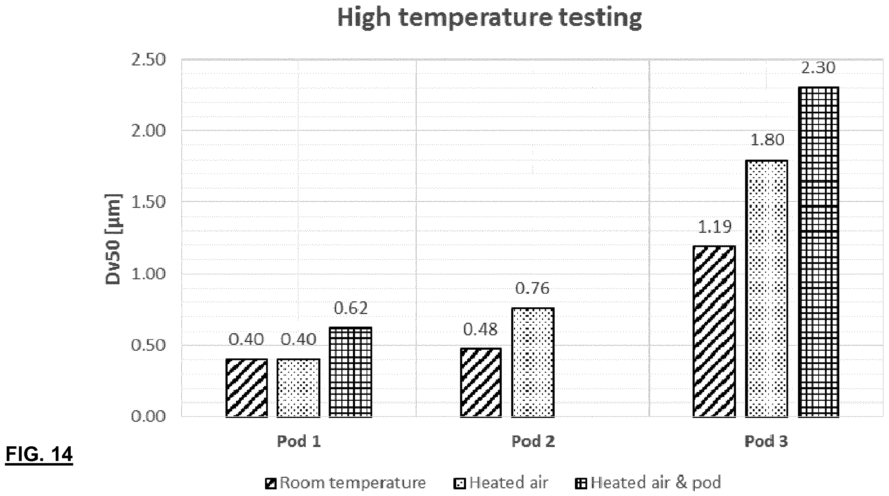

Figure 14 shows the high temperature testing results. Larger particle sizes were observed from all 3 pods when the temperature of inlet air increased from room temperature (23°C) to 50 °C. When the pods were heated as well, two of the three pods saw even larger particle size measurement results, while pod 2 was unable to be measured due to significant amount of leakage.

Without wishing to be bound by theory, the results are in line with the inventors' insight that control over the vapour cooling rate provides an important degree of control over the particle size of the aerosol. As reported above, the use of a slow air velocity can have the result of the formation of an aerosol with large Dv50. It is considered that this is due to slower air velocity allowing a slower cooling rate of the vapour.

Another conclusion related to laminar flow can also be explained by a cooling rate theory: laminar flow allows slow and gradual mixing between cold air and hot vapour, which means the vapour can cool down in slower rate when the airflow is laminar, resulting in larger particle size.

The results in Figure 14 further validate this cooling rate theory: when the inlet air has higher temperature, the temperature difference between hot vapour and cold air becomes smaller, which allows the vapour to cool down at a slower rate, resulting in larger particle size; when the pods were heated as well, this mechanism was exaggerated even more, leading to an even slower cooling rate and an even larger particle size.

4.5. Further consideration of vapour cooling rate

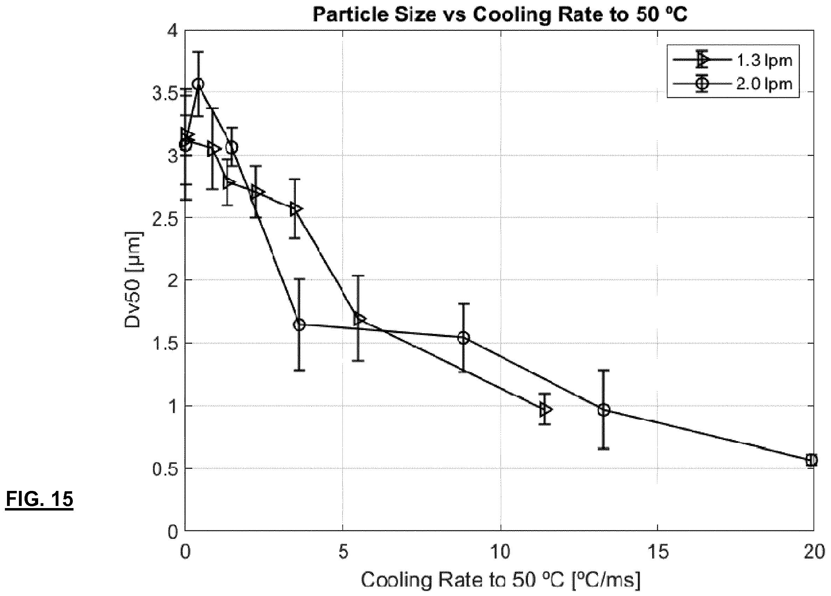

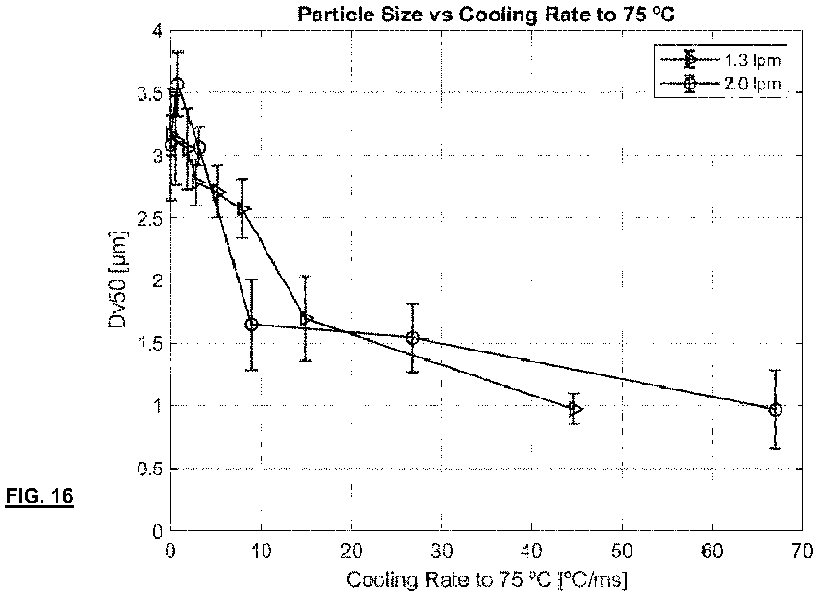

In section 3.3, the vapour cooling rates for each tube size and flow rate combination were obtained via multiphysics simulation. In Figure 15 and Figure 16, the particle size measurement results were plotted against vapour cooling rate to 50°C and 75°C, respectively.

The data in these graphs indicates that in order to obtain an aerosol with Dv50 larger than 1 $\mu$m, the apparatus should be operable to require more than 16 ms for the vapour to cool to 50°C, or an equivalent (simplified to an assumed linear) cooling rate being slower than 10 °C/ms. From an alternative viewpoint, in order to obtain an aerosol with Dv50 larger than 1 $\mu$m, the apparatus should be operable to require more than 4.5 ms for the vapour to cool to 75°C, or an equivalent (simplified to an assumed linear) cooling rate slower than 30 °C/ms.

Furthermore, in order to obtain an aerosol with Dv50 of 2 $\mu$m or larger, the apparatus should be operable to require more than 32 ms for the vapour to cool to 50°C, or an equivalent (simplified to an assumed linear) cooling rate being slower than 5 °C/ms. From an alternative viewpoint, in order to obtain an aerosol with Dv50 of 2 $\mu$m or larger, the apparatus should be operable to require more than 13 ms for the vapour to cool to 75°C, or an equivalent (simplified to an assumed linear) cooling rate slower than 10 °C/ms.

5. Conclusions of particle size experimental work

In this work, particle size (Dv50) of aerosols generated in a set of rectangular tubes was studied in order to decouple different factors (flow rate, air velocity, Reynolds number, tube size) affecting aerosol particle size. It is considered that air velocity is an important factor affecting particle size - slower air velocity leads to larger particle size. When air velocity was kept constant, the other factors (flow rate, Reynolds number, tube size) has low influence on particle size.

The role of turbulence was also investigated. It is considered that laminar air flow favours generation of large particles, and introducing turbulence deteriorates (reduces) the particle size.

Modelling methods were used to simulate the average air velocity, the maximum air velocity, and the turbulence intensity in the vicinity of the wick. A COMSOL model with three coupled physics has also been developed to obtain the vapour cooling rate.

All experimental and modelling results support a cooling rate theory that slower vapour cooling rate is a significant factor in ensuring larger particle size. Slower air velocity, laminar air flow and higher inlet air temperature lead to larger particle size, because they all allow vapour to cool down at slower rates.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

**Claims**

1. A smoking substitute apparatus for generating an aerosol, comprising:

   a housing;
   an air inlet and an outlet formed at the housing;
   a passage extending between the air inlet and the outlet, in use an air flow entering into the housing to flow along the passage to the outlet for inhalation by a user drawing on the apparatus;
   an aerosol generation chamber containing an aerosol generator being operable to generate an aerosol from an aerosol precursor; the aerosol generation chamber comprises at least one chamber outlet in fluid communication with the passage, the at least one chamber outlet permitting, in use,
   aerosol generated by the aerosol generator to be entrained into the air flow along the passage; and
   a bleed aperture opening into the aerosol generation chamber and configured to allow air to enter the aerosol generation chamber in an amount that contributes not more than 35% of the air flow at the outlet for inhalation by a user drawing on the apparatus.

2. The smoking substitute apparatus of claim 1, wherein the bleed aperture is configured to have a smaller hydraulic diameter to that of the passage.

3. The smoking substitute apparatus of claim 1 or claim 2, wherein the chamber outlet and the bleed aperture open at opposing ends of the aerosol generation chamber.

4. The smoking substitute apparatus of any one of the preceding claims, wherein the bleed aperture is in fluid communication with the passage through an auxiliary passage.

5. The smoking substitute apparatus of claim 4, wherein the auxiliary passage comprises an auxiliary flow constrictor for limiting the amount of air flow through the auxiliary passage.

6. The smoking substitute apparatus of claim 5, wherein the auxiliary flow constrictor comprises a narrowed section of the auxiliary passage.

7. The smoking substitute apparatus of claim 5 or claim 6, wherein the auxiliary flow constrictor comprises an adjustable flow constrictor for controlling the amount of air flow through the auxiliary passage.

8. The smoking substitute apparatus of any one of the preceding claims, wherein the bleed aperture comprises a one way valve, said one way valve being configured to allow the air flow to enter the aerosol generation chamber through the bleed aperture and to prevent leakage out of the aerosol generation chamber through the bleed aperture.

9. The smoking substitute apparatus of any one of the preceding claims, wherein the bleed aperture is configured to allow air to enter the aerosol generation chamber in an amount that contributes no more than any one of 30%, 25%, 20%, 15%, 10% and 5% of air flow at the outlet for inhalation by a user drawing on the apparatus.

10. The smoking substitute apparatus of any one of the preceding claims, wherein the bleed aperture has a hydraulic diameter in the range of any one of 0.01mm to 10mm, 0.01mm to 1mm, 0.1mm to 1mm and 0.3mm to 0.7mm.

11. The smoking substitute apparatus of any one of the preceding claims, wherein the bleed aperture comprises a plurality of bleed apertures distributed over a base of the aerosol generation chamber.

12. The smoking substitute apparatus of any one of the preceding claims, wherein the aerosol precursor comprises a liquid aerosol precursor, and wherein the aerosol generator comprises a heater configured to generate the aerosol by vaporising the liquid aerosol precursor.

13. The smoking substitute apparatus of any one of the preceding claims, wherein the smoking substitute apparatus is configured to generate an aerosol having a droplet size, $d_{50}$, of at least 1 $\mu$m.

14. A smoking substitute system for generating an aerosol, comprising:

   i) the smoking substitute apparatus of any one of the preceding claims; and
   ii) a main body configured to engage with the smoking substitute apparatus; wherein the main body comprises a controller and a power source configured to energise the aerosol generator.

15. A method of using the smoking substitute apparatus of any one of the claims 1 to 13, comprising:

   i) generating the aerosol with the aerosol generator; and
   ii) drawing on the apparatus to cause air to enter the aerosol generation chamber in an amount that contributes not more than 35% of the air flow at the outlet.

FIG. 1

1170

1162

1164

**FIG. 2**

1162   1164   1170

Constant depth
6 mm

Variable width
4.5mm - 50 mm

**FIG. 3**

$$I = 0.55\ \% \qquad I = 0.55\ \% \qquad I = 0.55\ \%$$

**FIG. 4A**

$$I = 0.77\,\%$$ $$I = 0.77\,\%$$ $$I = 0.77\,\%$$

FIG. 4B

$$I = 1.06\,\% \qquad I = 1.06\,\% \qquad I = 1.06\,\%$$

FIG. 4C

$$I = 1.34\,\%\qquad I = 1.34\,\%\qquad I = 1.34\,\%$$

**FIG. 4D**

**FIG. 5**

154x

160x

162x

156x

**FIG. 6**

FIG. 7

154z

160z

162z

156z

**FIG. 8A**

154z

160z

162z

**FIG. 8B**

FIG. 9

EP 3 794 976 A1

FIG. 10

EP 3 794 976 A1

**Particle Size vs Average Velocity**

FIG. 11

EP 3 794 976 A1

Particle Size vs Maximum Velocity

FIG. 12

**Particle Size vs Turbulence Intensity**

FIG. 13

EP 3 794 976 A1

## High temperature testing

**FIG. 14**

EP 3 794 976 A1

**FIG. 15**

**Particle Size vs Cooling Rate to 75 °C**

Legend: —▲— 1.3 lpm; —○— 2.0 lpm

X-axis: Cooling Rate to 75 °C [°C/ms]
Y-axis: Dv50 [μm]

**FIG. 16**

**FIG. 17**

**FIG. 18**

**FIG. 19**

**FIG. 20**

**FIG. 21A**

**FIG. 21B**

**FIG. 21C**

**FIG. 22**

Europäisches
Patentamt

European
Patent Office

Office européen
des brevets

# EUROPEAN SEARCH REPORT

Application Number

EP 19 19 8636

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/083635 A1 (PHILIP MORRIS PROD [CH]) 13 June 2013 (2013-06-13) | 1-4, 9-12,14, 15 | INV. A24F40/10 A24F40/40 A61M15/00 |
| Y | * figure 2 * | 5-8,13 | |
| Y | US 2014/202472 A1 (LEVITZ ROBERT [IL] ET AL) 24 July 2014 (2014-07-24) * paragraphs [0048], [0049]; figure 8 * | 5-7 | |
| Y | US 2017/325505 A1 (FORCE ERIC [CH] ET AL) 16 November 2017 (2017-11-16) * paragraphs [0037] - [0039], [0103], [0106] - [0108] * | 8 | |
| Y | WO 2016/040575 A1 (FONTEM HOLDINGS 1 BV [NL]; WENSLEY MARTIN [US] ET AL.) 17 March 2016 (2016-03-17) * page 4 - page 8; claim 4; figures 1-4 * | 13 | |

TECHNICAL FIELDS
SEARCHED     (IPC)

A24F
A61M

The present search report has been drawn up for all claims

| Place of search | Date of completion of the search | Examiner |
|---|---|---|
| Munich | 17 March 2020 | Schwertfeger, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 8636

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2020

| Patent document cited in search report | | | Publication date | Patent family member(s) | | | Publication date |
|---|---|---|---|---|---|---|---|
| WO 2013083635 | A1 | | 13-06-2013 | AR | 089124 | A1 | 30-07-2014 |
| | | | | WO | 2013083635 | A1 | 13-06-2013 |
| US 2014202472 | A1 | | 24-07-2014 | AU | 2012320060 | A1 | 22-05-2014 |
| | | | | BR | 112014008199 | A2 | 18-04-2017 |
| | | | | CA | 2851195 | A1 | 11-04-2013 |
| | | | | CN | 103917119 | A | 09-07-2014 |
| | | | | EP | 2750529 | A1 | 09-07-2014 |
| | | | | ES | 2667268 | T3 | 10-05-2018 |
| | | | | IL | 231800 | A | 31-10-2018 |
| | | | | JP | 6012741 | B2 | 25-10-2016 |
| | | | | JP | 2014533932 | A | 18-12-2014 |
| | | | | KR | 20140101728 | A | 20-08-2014 |
| | | | | NZ | 624454 | A | 26-08-2016 |
| | | | | PL | 2750529 | T3 | 28-09-2018 |
| | | | | RU | 2014117968 | A | 20-11-2015 |
| | | | | UA | 111630 | C2 | 25-05-2016 |
| | | | | US | 2014202472 | A1 | 24-07-2014 |
| | | | | US | 2017196271 | A1 | 13-07-2017 |
| | | | | WO | 2013050934 | A1 | 11-04-2013 |
| US 2017325505 | A1 | | 16-11-2017 | AR | 103016 | A1 | 12-04-2017 |
| | | | | CA | 2962756 | A1 | 23-06-2016 |
| | | | | CN | 107889450 | A | 06-04-2018 |
| | | | | EP | 3232841 | A1 | 25-10-2017 |
| | | | | JP | 2018500015 | A | 11-01-2018 |
| | | | | KR | 20170095188 | A | 22-08-2017 |
| | | | | RU | 2017124441 | A | 17-01-2019 |
| | | | | US | 2017325505 | A1 | 16-11-2017 |
| | | | | WO | 2016096780 | A1 | 23-06-2016 |
| WO 2016040575 | A1 | | 17-03-2016 | CN | 107847696 | A | 27-03-2018 |
| | | | | EP | 3191162 | A1 | 19-07-2017 |
| | | | | US | 2017246405 | A1 | 31-08-2017 |
| | | | | WO | 2016040575 | A1 | 17-03-2016 |

EPO FORM P0459

For more details about this annex : see Official Journal of the European Patent Office, No. 12/82